# EUROPEAN PATENT SPECIFICATION

(11) **EP 1 431 394 B1**
(45) Date of publication and mention of the grant of the patent: **02.08.2006**
(21) Application number: 02028536.7
(22) Date of filing: 20.12.2002
(51) Int. Cl.: C12N 15/82, A01H 11/00, C12P 21/02, C12N 9/10

(54) **Method to produce heterologous glycosylated proteins in bryophyte cells**
Verfahren zur Herstellung von glykosylierten Proteinen in Bryophyten Zellen
Procédé de production de protéines glycosylées dans des cellules de bryophytes

(43) Date of publication of application: 23.06.2004
(73) Proprietor: greenovation Biotech GmbH, 79111 Freiburg i.Br (DE)
(72) Inventor: Lienhart, Otmar, 79114 Freiburg (DE); Koprivova, Anna, Dr., Norwich, NR3 2RB (GB); Decker, Eva, Dr., 79104 Freiburg (DE); Reski, Ralf, Prof. Dr., 79254 Oberried-Zastler (DE); Gorr, Gilbert, Dr., 79112 Freiburg (DE); Stemmer, Christian, Dr., 79106 Freiburg (DE)
(74) Representative: Stürken, Joachim

(56) References cited:
- WO-A-01/25456
- WO-A-01/29242
- KOPRIVOVA ANNA ET AL: "Functional knockout of the adenosine 5'-phosphosulfate reductase gene in Physcomitrella patens revives an old route of sulfate assimilation." JOURNAL OF BIOLOGICAL CHEMISTRY, vol. 277, no. 35, 30 August 2002 (2002-08-30), pages 32195-32201, XP002245177 August 30, 2002 ISSN: 0021-9258
- SCHAEFER D G ET AL: "EFFICIENT GENE TARGETING IN THE MOSS PHYSCOMITRELLA PATENS" PLANT JOURNAL, BLACKWELL SCIENTIFIC PUBLICATIONS, OXFORD, GB, vol. 11, no. 6, June 1997 (1997-06), pages 1195-1206, XP000885764 ISSN: 0960-7412
- LEROUGE P ET AL: "N-glycoprotein biosynthesis in plants: recent developments and future trends" PLANT MOLECULAR BIOLOGY, NIJHOFF PUBLISHERS, DORDRECHT, NL, vol. 38, 1998, pages 31-48, XP002140796 ISSN: 0167-4412
- BAKKER HANS ET AL.: "Plant members of the alpha13/4-fucosyltransferase gene familiy encode an alpha1-4-fucosyltransferase, potentially involved in Lewis a biosynthesis, and two core alpha1-3-fucosyltransferase" FEBS LETTERS, vol. 507, 2001, pages 307-312,
- ANTJE VON SCHAEWEN ET AL.: "Isolation of a mutant of Arabidopsis plant that lacks N-acetyl glucosaminyl transferase I ans is unable to synthesize golgi-modified complex N-linked glycans" PLANT PHYSIOL, vol. 102, 2001, pages 1109-1118,

## Description

### Background

The present invention relates to a method for producing heterologous glycosylated proteins in bryophyte cells, such as in transformed *Physcomitrella patens* cells in culture. In particular, the method relates to a method for producing glycosylated proteins comprising animal glycosylation patterns, such as pharmaceutical proteins for use in mammals, including humans, in bryophyte cells such as *Physcomitrella patens* cells, the genetic material required therefore, such as DNA and RNA, vectors, host cells, methods of introduction of genetic material there into, and uses thereof.

In the past, heterologous proteins have been produced using a variety of transformed cell systems, such as those derived from bacteria, fungi, such as yeasts, insect, plant or mammalian cell lines (Kudo T. 1994, In: Y. Murooka and T. Amanaka (Eds.) Recombinant microbes for industrial and agricultural applications, pp. 291-299, Marcel Dekker, New York; Harashima S., Bioproc. Technol. 1994, 19: 137-158; Archer D.B. 1994, In: Y. Murooka and T. Amanaka (Eds.) Recombinant microbes for industrial and agricultural applications, pp. 373-393, Marcel Dekker, New York; Goosen M.F.A. 1993, In: M.F.A. Goosen, A. Baugulis and P. Faulkner (Eds.) Insect cell culture engineering, pp. 1-16, Marcel Dekker, New York; Hesse F. & Wagner R. ,Trends in Biotechnol. 2000, 18(4): 173-180).

Proteins produced in prokaryotic organisms may not be post-translationally modified in a similar manner to that of eukaryotic proteins produced in eukaryotic systems, e.g. they may not be glycosylated with appropriate sugars at particular amino acid residues, such as aspartic acid (N) residues (N-linked glycosylation). Furthermore, folding of bacterially-produced eukaryotic proteins may be inappropriate due to, for example, the inability of the bacterium to form cysteine disulfide bridges. Moreover, bacterially-produced recombinant proteins frequently aggregate and accumulate as insoluble inclusion bodies.

Eukaryotic cell systems are better suited for the production of glycosylated proteins found in various eukaryotic organisms, such as humans, since such cell systems may effect post-translational modifications, such as glycosylation of produced proteins. However, a problem encountered in eukaryotic cell systems which have been transformed with heterologous genes suitable for the production of protein sequences destined for use, for example, as pharmaceuticals, is that the glycosylation pattern on such proteins often acquires a native pattern, that is, of the eukaryotic cell system in which the protein has been produced: glycosylated proteins are produced that comprise non-animal glycosylation patterns and these in turn may be immunogenic and/or allergenic if applied in animals, including humans.

The use of recombinant glycoproteins produced by higher plants is limited by the plant-specific N-glycosylation that is acquired on such proteins. Compared to mammalian-derived glycoproteins, higher plant-specific glycoproteins contain two additional residues. Moreover, in higher plant glycoproteins terminal beta 1,4-galactose residues are not found, indicating that a beta 1,4-galactosyltransferase is not present in plants. Stable integration and expression of this enzyme in tobacco plants (Bakker et al. (2001) *Proc Natl Acad Sci USA,* 98 ,2899-2904) as well as in tobacco BY2 cells (Palacpac et al. (1999) *Proc Natl Acad Sci* USA 96,4692-4697) has been described. The recombinant human beta 1,4-galactosyltransferase was functional and proteins isolated from transgenic material exhibited terminal beta 1,4-galactose residues. Nevertheless, in higher plants it is not thought possible to suppress the activities of beta 1,2-xylosyltransferase and alpha 1,3-fucosyltransferase, the two enzymes that are considered responsible for transferring the additional, plant-specific residues. These residues are considered to be allergenic for humans (Garcia-Cassado et al. (1996) *Glycobiology* 6, 471-477; van Ree et al. 2000, *J. Biol. Chem.* 275, No.15,11451-11458). All data on plant-specific N-glycosylation has been generated in studies with higher plants.

WO 01/29242 A discloses higher plants that have been modified to express a mammalian beta-galactosyltransferase (GalT) in order to produce heterologous proteins with mammalian-like glycans. For this purpose it is suggested to reduce the plant own xylosyl or fucosyl transferase activity by antisense strategy using antisense constructs that were transformed independently and separately from each other.

The bryophyte, *Physcomitrella patens,* a haploid non-vascular land plant, can also be used for the production of recombinant proteins. For example, it is demonstrated in WO 01/25456 A and Schaefer, D.G. et al., 'Efficient gene targeting in the moss Physcomitrella patens', Plant J.,vol. 11, no. 6,p. 1195-1206 (1997), that bryophytes can successfully be used to produce functional heterologous proteins and that the technology is well-known and the targeting of a particular gene is feasible.

The life cycle of mosses is dominated by photoautotrophic gametophytic generation. The life cycle is completely different to that of the higher plants wherein the sporophyte is the dominant generation and there are notably many differences to be observed between higher plants and mosses.

The gametophyte of mosses is characterised by two distinct developmental stages. The protonema which develops via apical growth, grows into a filamentous network of only two cell types (chloronemal and caulonemal cells). The second stage, called the gametophore, differentiates by caulinary growth from a simple apical system. Both stages are photoautotrophically active. Cultivation of protonema without differentiation into the more complex gametophore has been shown for suspension cultures in flasks as well as for bioreactor cultures (WO 01/25456). Cultivation of fully differentiated and photoautrophically active multicellullar tissue containing only a few cell types is not described for higher plants. The genetic stability of the moss cell system provides an important advantage over plant cell cultures and the stability of photoautotrophically active bryophyte cultures has been confirmed (Rieck 1996, Strukturaufklarung und stereochemische Untersuchungen von Sesquiterpenen als Inhaltsstoffe atherischer Ole. Ph.D. thesis, Hamburg University). In cell cultures of higher plants the secondary metabolism is more differentiated and this results in differences in secondary metabolite profiles.

In addition, there are some important differences between mosses and higher plants on the biochemical level. Sulfate assimilation in *Physcomitrella patens* differs significantly from that in higher plants. The key enzyme of sulfate assimilation in higher plants is adenosine 5'-phosphosulfate reductase. In *Physcomitrella patens* an alternative pathway via phosphoadenosine 5'-phosphosulfate reductase co-exists (Koprivova et al. (2002) J. Biol. Chem. 277,32195-32201). This pathway has not been characterised in higher plants.

Furthermore, many members of the moss, algae and fern families produce a wide range of polyunsaturated fatty acids ( Dembitsky (1993) Prog. Lipid Res. 32, 281-356). For example, arachidonic acid and eicosapentaenoic acid are thought to be produced only by lower plants and not by higher plants. Some enzymes of the metabolism of polyunsaturated fatty acids, (delta 6-acyl-group desaturase) (Girke et al. (1998), *Plant J,* 15, 39-48) and a component of a delta 6 elongase (Zank et al. (2002) *Plant J* 31, 255-268), have been cloned from *Physcomitrella patens.* No corresponding genes have been found in higher plants. This fact appears to confirm that essential differences exist between higher plants and lower plants at the biochemical level.

Further differences are reflected in the regeneration of the cell wall. Protoplasts derived from higher plants regenerate new cell walls in a rapid manner, independently of the culture medium. Direct transfer of DNA via polyethylene glycol (PEG) into protoplasts of higher plants requires pre-incubation at 4 to 10°C to slow down the process of cell wall regeneration (US Patent 5508184). In contrast, cell wall regeneration of protoplasts derived from protonema of Physcomitrella is dependent on culture medium. Protoplasts can be cultivated without regeneration of the cell wall over long periods. Without the intention of being bound by theory, it appears that the secretion machinery of the moss protoplast, essential for cell wall regeneration and protein glycosylation, differs from that of higher plants. Moreover, *Physcomitrella patens* shows highly efficient homologous recombination in its nuclear DNA, a unique feature for plants, which enables directed gene disruption (Girke et al. (1998) *Plant J*, 15, 39-48; Strepp et al. (1998) *Proc Natl Acad Sci* USA 95,4368-4373; Koprivova (2002) J. Biol. Chem.277, 32195-32201; reviewed by Reski (1999) *Planta* 208, 301-309; Schaefer and Zryd (2001) *Plant Phys* 127, 1430-1438; Schaefer (2002) *Annu. Rev. Plant Biol*.53, 477-501) further illustrating fundamental differences to higher plants. However, the use of this mechanism for altering glycosylation patterns has proven to be problematic, as shown herein in the examples. Disruption of N-acetylglucosaminyltransferase I (GNT1) in *Physcomitrella patens* resulted in the loss of the specific transcript but only in minor differences of the N-glycosylation pattern. These results were in direct contrast to the loss of Golgi-modified complex glycans in a mutant *Arabidopsis thaliana* plant lacking GNT1 observed by von Schaewen et al. (1993) *Plant Physiol* 102, 1109-1118). Thus, the knockout in *Physcomitrella patens* did not result in the expected modification of the N-glycosylation pattern.

Although the knockout strategy was not successful for GNT1, the present inventors attempted to knock out the beta 1,2-xylosyltransferase (XylT) and alpha 1,3-fucosyltransferase (FucT) in *Physcomitrella patens*. Specific transcripts could not be detected in the resulting plants. Surprisingly, the N-linked glycans isolated from the transgenic plants were found to be modified in the desired manner. No 1,3 linked fucosyl residues could be detected on N-linked glycans of FucT knockout plants and no 1,2 linked xylosyl residues could be detected on N-linked glycans of XylT knockout plants. The isolated transgenic lines showed normal growth which is surprising considering that plant specific glycosylation is highly conserved and therefore would be expected to be significant for function. Double knockouts should therefore have been expected to have had a detrimental effect on the growth of the moss. In addition, compensating were expected but surprisingly were not apparent. Moreover, the double knockout of FucT and XylT resulted in modified N-linked glycans without detectable 1,3 linked fucosyl and 1,2 linked xylosyl residues.

Integration of the human beta 1,4-galactosyltransferase into the genome of a double knockout *Physcomitrella patens* plant resulted in a mammalian-like N-linked glycosylation pattern without the plant specific fucosyl and xylosyl residues and with mammalian-like terminal 1,4 galactosyl residues. The galactosyltransferase was found to be active. Such a modification of N-linked glycans without loss of viability was not expected because N-glycosylation is very complex and well regulated. It is not only dependent on developmental stages (for plants: Elbers et al. (2001) *Plant Phys* 126, 1314-1322) but also dependent on culture conditions (for mammalian cell culture: Hills et al. (2001) Biotechnol. Bioeng. 75, 239-251).

It is an object of the present invention to provide a more efficient method of producing animal proteins comprising animal glycosylation patterns, and in particular, glycosylated human proteins comprising human glycosylation patterns thereon. It is a further object to provide an efficient process for the production of heterologous animal proteins comprising animal glycosylation patterns, particularly human proteins comprising human glycosylation patterns in bryophytes, such as *Physcomitrella patens.*

These and other objects will become apparent from the following description and examples provided herein.

### Detailed Description

According to the present invention there is provided a transformed bryophyte cell that comprises a double knockout of fucosyl transferase and xylosyl transferase that results in modified N-linked glycans without detectable 1,3-linked fucosyl and 1,2-linked xylosyl residues.

The bryophyte cell of the invention is one from a moss selected from the group mosses including liverworts, of species from the genera *Physcomitrella, Funaria, Sphagnum, Ceratodon, Marchantia* and *Sphaerocarpos.* The bryophyte cell is preferably from *Physcomitrella patens.*

The bryophyte cell, such as a *Physcomitrella patens* cell, can be any cell suitable for transformation according to methods of the invention as described herein, and may be a moss protoplast cell, a cell found in protonema tissue or other cell type. Indeed, the skilled addressee will appreciate that moss plant tissue comprising populations of transformed bryophyte cells according to the invention, such as transformed protonemal tissue also forms an aspect of the present invention.

"Dysfunctional" as used herein means that the nominated transferase nucleotide sequences of fucosyl transferase (fucT) and xylosyl transferase (xylT) are substantially incapable of encoding mRNA that codes for functional fucT and xylT proteins that are capable of modifying plant N-linked glycans with plant-like glycoslation patterns comprising 1,3 linked fucosyl and 1,2 linked xylosyl residues. In a preferment, the dysfunctional fucT and xylT plant transferase nucleotide sequences comprise targeted insertions of exogenous nucleotide sequences into endogenous, that is genomic, native fucT and xylT genes comprised in the nuclear bryophyte genome (whether it is a truly native bryophyte genome, that is in bryophyte cells that have not been transformed previously by man with other nucleic acid sequences, or in a transformed nuclear bryophyte genome in which nucleic acid sequence insertions have been made previously of desired nucleic acid sequences) which substantially inhibits or represses the transcription of mRNA coding for functional fucT and xylT transferase activity.

Bryophyte cells of the invention or ancestors thereof may be any which have been transformed previously with heterologous genes of interest that code for primary sequences of proteins of interest which are glycosylated with mammalian glycosylation patterns as described herein. Preferably, the glycosylation patterns are of the human type. Alternatively, the bryophyte cell may be transformed severally, that is, simultaneously or over time with nucleotide sequences coding for at least a primary protein sequence of interest, typically at least a pharmaceutical protein of interest for use in humans or mammals such as livestock species including bovine, ovine, equine and porcine species, that require mammalian glycosylation patterns to be placed on them in accordance with the methods of the invention as described herein. Such pharmaceutical glycoproteins for use in mammals, including man include but are not limited to proteins such as insulin, preproinsulin, VEGF, proinsulin, glucagon, interferons such as alpha-interferon, beta-interferon, gamma-interferon, blood-clotting factors selected from Factor VII, VIII, IX, X, XI, and XII, fertility hormones including luteinising hormone, follicle stimulating hormone, growth factors including epidermal growth factor, platelet-derived growth factor, granulocyte colony stimulating factor and the like, prolactin, oxytocin, thyroid stimulating hormone, adrenocorticotropic hormone, calcitonin, parathyroid hormone, somatostatin, erythropoietin (EPO), enzymes such as betaglucocerebrosidase, haemoglobin, serum albumin, collagen, fusion proteins such as the fusion protein of TNF alpha receptor ligand binding domain with Fc portion of IgG and the like. Furthermore, the method of the invention can be used for the production of antibodies such as specific monoclonal antibodies or active fragments thereof.

In a preferment, there is provided a transformed bryophyte cell that comprises a double knockout of fucosyl transferase and xylosyl transferase that results in modified N-linked glycans without detectable 1,3-linked fucosyl and 1,2-linked xylosyl residue and iii) a nucleotide sequence operably linked to an exogenous promoter that drives expression in the said bryophyte cell wherein said nucleotide sequence encodes a functional mammalian galactosyl transferase that is expressed in the bryophyte cell.

In a further preferment, the mammalian galactosyl transferase nucleotide sequence is a beta-1,4 galactosyl transferase (beta-1,4 galT) and most preferably is a human beta-1,4 galactosyl transferase nucleotide sequence. The skilled addressee will appreciate that the beta-1,4 galT nucleotide sequence may be a cDNA sequence or a genomic DNA sequence and may comprise a degeneratively equivalent nucleotide beta-1,4 galT sequence as long as the N-glycan glycosylation pattern on any desired glycosylated exogenous protein produced in the transformed bryophyte cells or bryophyte tissue of the invention is substantially mammalian in pattern, if not completely mammalian in pattern, and most preferably, where appropriate, is human in pattern.
Detailed information on the culturing of mosses which are suitable for use in the invention, such as *Leptobryum pyriforme* and *Sphagnum magellanicum* in bioreactors, is known in the prior art (see, for example, E. Wilbert, "Biotechnological studies concerning the mass culture of mosses with particular consideration of the arachidonic acid metabolism", Ph.D. thesis, University of Mainz (1991); H. Rudolph and S. Rasmussen, Studies on secondary metabolism of Sphagnum cultivated in bioreactors, Crypt. Bot., 3, pp. 67-73 (1992)). Especially preferred for the purposes of the present invention is the use of *Physcomitrella patens,* since molecular biology techniques are practised on this organism (for a review see R. Reski, Development, genetics and molecular biology of mosses, Bot. Acta, 111, pp. 1-15 (1998)).

Suitable transformation systems have been developed for the biotechnological exploitation of *Physcomitrella* for the production of heterologous proteins. For example, successful transformations have been carried out by direct DNA transfer into protonema tissue using particle guns. PEG-mediated DNA transfer into moss protoplasts has also been successfully achieved. The PEG-mediated transformation method has been described many times for *Physcomitrella patens* and leads both to transient and to stable transformants (see, for example, K. Reutter and R. Reski, Production of a heterologous protein in bioreactor cultures of fully differentiated moss plants, Pl. Tissue culture and Biotech., 2, pp. 142-147 (1996)).

In a further embodiment of the present invention there is provided a method of producing at least a bryophyte cell that comprises a double knockout of fucosyl transferase and xylosyl transferase that results in modified N-linked glycans without detectable 1,3-linked fucosyl and 1,2-linked xylosyl residues, that comprises introducing into the said cell i) a first nucleic acid sequence that is specifically targeted to an endogenous fucosyl transferase nucleotide sequence and ii) introducing into the said cell a second nucleic acid sequence that is specifically targeted to an endogenous xylosyl transferase nucleotide sequence.

Accordingly, the present invention also provides a set of nucleic acid vectors suitable for producing at least a transformed bryophyte cell that comprises a double knockout of fucosyl transferase and xylosyl transferase that results in modified N-linked glycans without detectable 1,3-linked fucosyl and 1,2-linked xylosyl residues, wherein said set of nucleic acid vectors comprises i) a first nucleic acid sequence that is specifically targeted to an endogenous fucosyl transferase nucleotide sequence and ii) a second nucleic acid sequence that is specifically targeted to an endogenous xylosyl transferase nucleotide sequence.

According to a preferred embodiment, said set of nucleic acid vectors further includes a polynucleotide that encodes a glycosylated polypeptide. According to another preferred embodiment, said set of nucleic acid vectors further includes a polynucleotide that encodes a functional mammalian glycosyl transferase, preferably a mammalian galactosyl transferase, with a polynucleotide that encodes a human beta-1,4 galactosyl transferase being most preferred.

On the basis of said set of nucleic acid vectors, host cells containing such a set of vectors, methods of producing such host cells including incorporating said set of nucleic acid vectors into the cell by means of transformation, uses of the set of nucleic acid vectors in the production of a transgenic bryophyte cell, are also provided by the present invention.

The skilled addressee will appreciate that the order of introduction of said first and second transferase nucleic acid sequences into the bryophyte cell is not important: it can be performed in any order. The first and second nucleic acid sequences can be targeted to specific portions of the endogenous, native fucT and xylT genes located in the nuclear genome of the bryophyte cell defined by specific restriction enzyme sites thereof, for example, according to the examples as provided herein. By specifically targeting the sequences of the native fucT and xylT transferase genes with nucleotide sequences that specifically integrate with the target native transferase genes of interest, the expression of the said sequences is substantially impaired if not completely disrupted.

In a preferred embodiment of the present invention there is provided a method of producing at least a heterologous or exogenous glycosylated mammalian protein in a transformed bryophyte cell that comprises:
i) introducing into said cell a first isolated nucleic acid that comprises a first nucleic acid sequence that is specifically targeted to an endogenous fucosyl transferase nucleotide sequence; and
ii) introducing into said cell a second nucleic acid sequence that is specifically targeted to an endogenous xylosyl transferase nucleotide sequence; and
iii) introducing into said cell a third isolated nucleic acid sequence that comprises nucleic acid operably linked to an exogenous promoter that drives expression in a bryophyte cell wherein said nucleic acid encodes at least one mammalian galactosyl transferase polypeptide.

In this embodiment of the invention the at least one mammalian galactosyl transferase polypeptide is preferably a beta-1,4 galactosyl transferase (beta-1,4 galT) and most preferably is a human beta-1,4 galactosyl transferase nucleotide sequence.

Preferably all glycosylated mammalian proteins mentioned hereinabove are of the human type. Other proteins that are contemplated for production in the present invention include proteins for use in veterinary care and may correspond to animal homologues of the human proteins mentioned herein.

An exogenous promoter is one that denotes a promoter that is introduced in front of a nucleic acid sequence of interest and is operably associated therewith. Thus an exogenous promoter is one that has been placed in front of a selected nucleic acid component as herein defined and does not consist of the natural or native promoter usually associated with the nucleic acid component of interest as found in wild type circumstances. Thus a promoter may be native to a bryophyte cell of interest but may not be operably associated with the nucleic acid of interest in front in wild-type bryophyte cells. Typically, an exogenous promoter is one that is transferred to a host bryophyte cell from a source other than the host cell.

The cDNA's encoding the galT proteins, the glycosylated and the mammalian proteins as described herein contain at least one type of promoter that is operable in a bryophyte cell, for example, an inducible or a constitutive promoter operatively linked to a galT nucleic acid sequence and/or second nucleic acid sequence for a glycosylated mammalian protein as herein defined and as provided by the present invention. As discussed, this enables control of expression of the gene(s).

The term "inducible" as applied to a promoter is well understood by those skilled in the art. In essence, expression under the control of an inducible promoter is "switched on" or increased in response to an applied stimulus (which may be generated within a cell or provided exogenously). The nature of the stimulus varies between promoters. Some inducible promoters cause little or undetectable levels of expression (or no expression) in the absence of the appropriate stimulus. Other inducible promoters cause detectable constitutive expression in the absence of the stimulus. Whatever the level of expression is in the absence of the stimulus, expression from any inducible promoter is increased in the presence of the correct stimulus. The preferable situation is where the level of expression increases upon application of the relevant stimulus by an amount effective to alter a phenotypic characteristic. Thus an inducible (or "switchable") promoter may be used which causes a basic level of expression in the absence of the stimulus which level is too low to bring about a desired phenotype (and may in fact be zero). Upon application of the stimulus, expression is increased (or switched on) to a level, which brings about the desired phenotype.

As alluded to herein, bryophyte expression systems are also known to the man skilled in the art. A bryophyte promoter, in particular a *Physcomitrella patens* promoter, is any DNA sequence capable of binding a host DNA-dependent RNA polymerase and initiating the downstream (3') transcription of a coding sequence (e.g. structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A bryophyte promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

The skilled addressee will appreciate that bryophyte promoter sequences encoding enzymes in bryophyte metabolic pathways can provide particularly useful promoter sequences.

In addition, synthetic promoters which do not occur in nature may also function as bryophyte promoters. For example, UAS sequences of one byrophyte promoter may be joined with the transcription activation region of another bryophyte promoter, creating a synthetic hybrid promoter. An example of a suitable promoter is the one used in the TOP 10 expression system for *Physcomitrella patens* by Zeidler et al. (1996) Plant. Mol. Biol. 30, 199-205). Furthermore, a bryophyte promoter can include naturally occurring promoters of non-bryophyte origin that have the ability to bind a bryophyte DNA-dependent RNA polymerase and initiate transcription. Examples of such promoters include those described, inter alia, the rice P-Actin 1 promoter and the Chlamydomonas RbcS promoter (Zeidler et al. (1999) J. Plant Physiol. 154, 641-650), Cohen et al., Proc. Natl. Acad. Sci. USA, 77: 1078, 1980; Henikoff et al., Nature, 283: 835, 1981; Hollenberg et al., Curr. Topics Microbiol. Immunol., 96: 119, 198 1; Hollenberg et al., "The Expression of Bacterial Antibiotic Resistance Genes in the Yeast *Saccharomyces cerevisiae",* in: Plasmids of Medical, Environmental and Commercial Importance (eds. K. N. Timms and A. Puhler), 1979; Mercerau-Puigalon et al., Gene, 1 1: 163, 1980; Panthier et al., Curr. Genet., 2: 109, 1980.

A DNA molecule may be expressed intracellularly in bryophytes. A promoter sequence may be directly linked with the DNA molecule, in which case the first amino acid at the N-terminus of the recombinant protein will always be a methionine, which is encoded by the AUG start codon on the mRNA. If desired, methionine at the N-terminus may be cleaved from the protein by in vitro incubation with cyanogen bromide.

Alternatively, foreign proteins can also be secreted from the bryophyte cell into the growth media by creating chimeric DNA molecules that encode a fusion protein comprised of a leader sequence fragment that provides for secretion in or out of bryophyte cells of the foreign protein. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell.

DNA encoding suitable signal sequences can be derived from genes for secreted bryophyte proteins, such as leaders of non-bryophyte origin, such as aVEGF leader, exist that may also provide for secretion in bryophyte cells.

Transcription termination sequences that are recognized by and functional in bryophyte cells are regulatory regions located 3' to the translation stop codon, and thus together with the promoter flank the coding sequence. These sequences direct the transcription of an mRNA which can be translated into the polypeptide encoded by the DNA. An example of a suitable termination sequence that works in *Physcomitrella patens* is the termination region of Cauliflower mosaic virus.

Typically, the components, comprising a promoter, leader (if desired), coding sequence of interest, and transcription termination sequence, are put together into expression constructs of the invention. Expression constructs are often maintained in a DNA plasmid, which is an extrachromosomal element capable of stable maintenance in a host, such as a bacterium. The DNA plasmid may have two origins of replication, thus allowing it to be maintained, for example, in a bryophyte for expression and in a prokaryotic host for cloning and amplification. Generally speaking it is sufficient if the plasmid has one origin of replication for cloning and amplification in a prokaryotic host cell. In addition, a DNA plasmid may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Either a high or low copy number vector may be selected, depending upon the effect of the vector and the foreign protein on the host (see, e.g., Brake et al., supra).

Alternatively, the expression constructs can be integrated into the bryophyte genome with an integrating vector. Integrating vectors usually contain at least one sequence homologous to a bryophyte chromosome that allows the vector to integrate, and preferably contain two homologous sequences flanking the expression construct. An integrating vector may be directed to a specific locus in moss by selecting the appropriate homologous sequence for inclusion in the vector as described and exemplified herein. One or more expression constructs may integrate. The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of bryophyte cells that have been transformed.

Selectable markers may include biosynthetic genes that can be expressed in the moss host, such as the G418 or hygromycin B resistance genes, which confer resistance in bryophyte cells to G418 and hygromycin B, respectively. In addition, a suitable selectable marker may also provide bryophyte cells with the ability to grow in the presence of toxic compounds, such as metal.

Alternatively, some of the above-described components can be put together into transformation vectors. Transformation vectors are usually comprised of a selectable marker that is either maintained in a DNA plasmid or developed into an integrating vector, as described above.

Methods of introducing exogenous DNA into bryophyte cells are well-known in the art, and are described inter alia by Schaefer D. G. "Principles and protocols for the moss *Physcomitrella patens",* (May 2001) Institute of Ecology, Laboratory of Plant Cell Genetics, University of Lausanne Didier.Schaefer@ie-pc.unil.ch; Reutter K. and Reski R., Plant Tissue Culture and Biotechnology September 1996, Vol.2, No.3; Zeidler M et al., (1996), Plant Molecular Biology 30:199-205.

Those skilled in the art are well able to construct vectors and design protocols for recombinant nucleic acid sequence or gene expression as described above. Suitable vectors can be chosen or constructed, containing appropriate regulatory sequences, including promoter sequences, terminator fragments, polyadenylation sequences, enhancer sequences, marker genes and other sequences as appropriate. For further details see, for example, *Molecular Cloning: a Laboratory Manual:* 2nd edition, Sambrook *et al,* 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in *Current Protocols in Molecular Biology,* Second Edition, Ausubel et al. eds., John Wiley & Sons, 1992. The disclosures of Sambrook et al. and Ausubel et al. are incorporated herein by reference.

Naturally, the skilled addressee will appreciate that each nucleic acid sequence coding for the appropriate galT and polypeptide to be glycosylated will be under regulatory control of its own exogenous promoter and terminator. When two or more target proteins are destined to be produced from a single carrier RNA it is preferable if they are able to be readily separated, for example by binding to different protein-specific antibodies (monoclonal or polyclonal) in the harvesting phase of the bryophyte cell culture system.

As described above, selectable genetic markers may facilitate the selection of transgenic bryophyte cells and these may consist of chimaeric genes that confer selectable phenotypes as alluded to herein.

When introducing selected glycosyl transferase nucleic acid sequences and polypetide sequences for glycosylation into a bryophyte cell, certain considerations must be taken into account, well known to those skilled in the art. The nucleic acid(s) to be inserted should be assembled within a construct, which contains effective regulatory elements, which will drive transcription. There must be available a method of transporting the construct into the cell. Once the construct is within the cell membrane, integration into the endogenous chromosomal material either will or will not occur.

The invention further encompasses a host cell transformed with vectors or constructs as set forth above, especially a bryophyte or a microbial cell. Thus, a host cell, such as a bryophyte cell, including nucleotide sequences of the invention as herein indicated is provided. Within the cell, the nucleotide sequence may be incorporated within the chromosome.

Also according to the invention there is provided a bryophyte cell having incorporated into its genome at least a nucleotide sequence, particularly heterologous nucleotide sequences, as provided by the present invention under operative control of regulatory sequences for control of expression as herein described. The coding sequence may be operably linked to one or more regulatory sequences which may be heterologous or foreign to the nucleic acid sequences employed in the invention, such as not naturally associated with the nucleic acid sequence(s) for its(their) expression. The nucleotide sequence according to the invention may be placed under the control of an externally inducible promoter to place expression under the control of the user. A further aspect of the present invention provides a method of making such a bryophyte cell, particularly a *Physcomitrella patens* cell involving introduction of nucleic acid sequence(s) contemplated for use in the invention or at least a suitable vector including the sequence(s) contemplated for use in the invention into a bryophyte cell and causing or allowing recombination between the vector and the bryophyte cell genome to introduce the said sequences into the genome. The invention extends to bryophyte cells, particularly *Physcomitrella patens* cells containing a GalT nucleotide and/or a nucleotide sequence coding for a polypeptide sequence destined for the addition of a mammalian glycosylation pattern thereto and suitable for use in the invention as a result of introduction of the nucleotide sequence into an ancestor cell.

The term "heterologous" may be used to indicate that the gene/sequence of nucleotides in question have been introduced into bryophyte cells or an ancestor thereof, using genetic engineering, ie by human intervention. A transgenic bryophyte cell, i.e. transgenic for the nucleotide sequence in question, may be provided. The transgene may be on an extra-genomic vector or incorporated, preferably stably, into the genome. A heterologous gene may replace an endogenous equivalent gene, ie one that normally performs the same or a similar function, or the inserted sequence may be additional to the endogenous gene or other sequence. An advantage of introduction of a heterologous gene is the ability to place expression of a sequence under the control of a promoter of choice, in order to be able to influence expression according to preference. Nucleotide sequences heterologous, or exogenous or foreign, to a bryophyte cell may be non-naturally occurring in cells of that type, strain or species. Thus, a nucleotide sequence may include a coding sequence of or derived from a particular type of bryophyte cell, such as a *Physcomitella patens* cell, placed within the context of a bryophyte cell of a different type or species. A further possibility is for a nucleotide sequence to be placed within a bryophyte cell in which it or a homologue is found naturally, but wherein the nucleotide sequence is linked and/or adjacent to nucleic acid which does not occur naturally within the cell, or cells of that type or species or strain, such as operably linked to one or more regulatory sequences, such as a promoter sequence, for control of expression. A sequence within a bryophyte or other host cell may be identifiably heterologous, exogenous or foreign.

The present invention also encompasses the desired polypeptide expression product of the combination of nucleic acid molecules according to the invention as disclosed herein or obtainable in accordance with the information and suggestions herein. Also provided are methods of making such an expression product by expression from nucleotide sequences encoding therefore under suitable conditions in suitable host cells e.g. E.coli. Those skilled in the art are well able to construct vectors and design protocols and systems for expression and recovery of products of recombinant gene expression.

A polypeptide according to the present invention may be an allele, variant, fragment, derivative, mutant or homologue of the(a) polypeptides as mentioned herein. The allele, variant, fragment, derivative, mutant or homologue may have substantially the same function of the polypeptides alluded to above and as shown herein or may be a functional mutant thereof. In the context of pharmaceutical proteins as described herein for use in humans, the skilled addressee will appreciate that the primary sequence of such proteins and their glycosylation pattern will mimick or preferably be identical to that found in humans.

"Homology" in relation to an amino acid sequence of the invention may be used to refer to identity or similarity, preferably identity. As noted already above, high level of amino acid identity may be limited to functionally significant domains or regions, e.g. any of the domains identified herein.

In particular, homologues of the particular bryophyte-derived polypeptide sequences provided herein, are provided by the present invention, as are mutants, variants, fragments and derivatives of such homologues. Such homologues are readily obtainable by use of the disclosures made herein. Naturally, the skilled addressee will appreciate that homologues of the glycosylated protein sequences per se, other than those homologues that due to the degeneracy of the genetic code give rise to amino acid sequences that are true copies (i.e. 100% identical) of the mammalian proteins of interest, and especially of human proteins of interest, are encompassed within the present invention. Thus the present invention also extends to polypeptides which include amino acid sequences with GalT function as defined herein and as obtainable using sequence information as provided herein. The GalT homologues may at the amino acid level have homology, that is identity, with the amino acid sequences described in the prior art as described herein i.e. under the database accession numbers provided in the examples section, preferably at least about 50%, or at least 55%, or at least about 60%, or at least about 65%, or at least about 70%, or at least about 75%, or at least about 80% homology, or at least about 85 %, or at least about 88% homology, or at least about 90% homology and most preferably at least about 95% or greater homology provided that such proteins have a GalT activity that fits within the context of the present invention.

In certain embodiments, an allele, variant, derivative, mutant derivative, mutant or homologue of the specific sequence may show little overall homology, say about 20%, or about 25%, or about 30%, or about 35%, or about 40% or about 45%, with the specific sequence. However, in functionally significant domains or regions, the amino acid homology may be much higher. Putative functionally significant domains or regions can be identified using processes of bioinformatics, including comparison of the sequences of homologues.

Functionally significant domains or regions of different polypeptides may be combined for expression from encoding nucleic acid as a fusion protein. For example, particularly advantageous or desirable properties of different homologues may be combined in a hybrid protein, such that the resultant expression product, with GalT function, may include fragments of various parent proteins, if appropriate.

Similarity of amino acid sequences may be as defined and determined by the TBLASTN program, of Altschul *et al.* (1990) *J. Mol. Biol.* **215:** 403-10, which is in standard use in the art. In particular, TBLASTN 2.0 may be used with Matrix BLOSUM62 and GAP penalties: existence: 11, extension: 1. Another standard program that may be used is BestFit, which is part of the Wisconsin Package, Version 8, September 1994, (Genetics Computer Group, 575 Science Drive, Madison, Wisconsin, USA, Wisconsin 53711). BestFit makes an optimal alignment of the best segment of similarity between two sequences. Optimal alignments are found by inserting gaps to maximize the number of matches using the local homology algorithm of Smith and Waterman (*Adv. Appl. Math.* (1981) 2: 482-489). Other algorithms include GAP, which uses the Needleman and Wunsch algorithm to align two complete sequences that maximizes the number of matches and minimizes the number of gaps. As with any algorithm, generally the default parameters are used, which for GAP are a gap creation penalty = 12 and gap extension penalty = 4. Alternatively, a gap creation penalty of 3 and gap extension penalty of 0.1 may be used. The algorithm FASTA (which uses the method of Pearson and Lipman (1988) *PNAS USA* **85**: 2444-2448) is a further alternative.

Use of either of the terms "homology" and "homologous" herein does not imply any necessary evolutionary relationship between compared sequences, in keeping for example with standard use of terms such as "homologous recombination" which merely requires that two nucleotide sequences are sufficiently similar to recombine under the appropriate conditions.

It is to be understood that the teaching of all references cited herein is incorporated into the teaching of the specification.

### Examples

### Methods and Materials

### Plant material

The wild-type strain of *Physcomitrella patens* (Hedw.) B.S.G. characterised by Reski et al. (1994) Genome analysis of the moss *Physcomitrella patens* (Hedw.) B.S.G.. *Mol Gen Genet* **244,** 352-359) was used. It is a subculture of strain 16/14 which was collected by H.L.K. Whitehouse in Gransden Wood, Huntingdonshire, UK and was propagated by Engel (1968) Am *J Bot* 55, 438-446.).

### Construction of pRT101VEGF C3

Human vascular endothelial growth factor 121 (VEGF₁₂₁) cDNA without leader sequence was excised as an *Nde*I-*Sal*I fragment from pCYTEXP-VEGF₁₂₁ (available from GBF, Braunschweig, Germany). This fragment was blunt-ended by the Klenow reaction and introduced into pRT101 (Töpfer et al. (1987) *NAR* 15, 589) at the *Sma*I restriction site to form plasmid pRT101VEGF C3. In this construct, the VEGF₁₂₁ cDNA without leader sequence was placed downstream of the CaMV 35 S promoter and terminated by the CaMV terminator (Gorr (1999) Biotechnologische Nutzung von Physcomitrella patens (Hedw.) B.S.G.. Ph.D. thesis, Hamburg University).

### Construction of pRT101TPVEGF C3

The sequence for VEGF signal peptide (sorting signal for secretion) was cloned into pRT101VEGF C3. The signal peptide cDNA was amplified from the plasmid pRT101 P21 (Gorr (1999),supra) using the 5' primer MoB323 5'- ATA CTC GAG GAA GAT GAA CTT TTC TGC CTG TCT TGG -3' (SEQ ID NO 1) containing an *Xho*I restriction side and 3' primer MoB349 5'- CTG CCA TGG GTG CAG CCT GGG ACC AC -3' (SEQ ID NO 2) containing a *Nco*I restriction side. The amplified DNA was digested with XhoI and NcoI and ligated into pRT101VEGF C3 (*Xho*I / *Nco*I digested) resulting in pRT101TPVEGF C3. The resulting plasmid contained the coding sequences for the VEGF signal peptide and VEGF₁₂₁ in frame under the control of the CaMV 35 S promoter.

### Standard culture conditions

Plants were grown axenicallly under sterile conditions in plain inorganic liquid modified Knop medium (1000 mg/l Ca(NO₃)₂ x 4H₂O 250 mg/l·KCl, 250 mg/l KH₂PO4, 250 mg/l MgSO₄ x 7 H₂O and 12.5 mg/l FeSO₄ X 7 H₂O; pH 5.8 (Reski and Abel (1985) *Planta* 165, 354-358). Plants were grown in 500 ml Erlenmeyer flasks containing 200 ml of culture medium and flasks were shaken on a Certomat R shaker (B.Braun Biotech International, Germany) set at 120 rpm. Conditions in the growth chamber were 25 +/- 3°C and a light-dark regime of 16:8 h. The flasks were illuminated from above by two fluorescent tubes (Osram L 58 W / 25) providing 35 micromols⁻¹m⁻². The cultures were subcultured once a week by disintegration using an Ultra-Turrax homogenizer (IKA, Staufen, Germany) and inoculation of two new 500 ml Erlenmeyer flasks containing 100 ml fresh Knop medium.

### Protoplast isolation

After filtration the moss protonemata were preincubated in 0.5 M mannitol. After 30 min, 4 % Driselase (Sigma, Deisenhofen, Germany) was added to the suspension. Driselase was dissolved in 0.5 M mannitol (pH 5.6-5.8), centrifuged at 3600 rpm for 10 min and sterilised by passage through a 0.22 microm filter (Millex GP, Millipore Corporation, USA). The suspension, containing 1% Driselase (final concentration), was incubated in the dark at RT and agitated gently (best yields of protoplasts were achieved after 2 hours of incubation) (Schaefer, "Principles and protocols for the moss *Physcomitrella patens",* (May 2001) Institute of Ecology, Laboratory of Plant Cell Genetics, University of Lausanne Didier.Schaefer@ie-pc.unil.ch). The suspension was passed through sieves (Wilson, CLF, Germany) with pore sizes of 100 microm and 50 microm. The suspension was centrifuged in sterile centrifuge tubes and protoplasts were sedimented at RT for 10 min at 55 g (acceleration of 3; slow down at 3; Multifuge 3 S-R, Kendro, Germany) (Schaefer, supra). Protoplasts were gently resuspended in W5 medium (125 mM CaCl₂ x 2H₂O; 137 mM NaCl; 5.5 mM glucose; 10 mM KCl; pH 5.6; 660-680 mOsm; sterile filtered). The suspension was centrifuged again at RT for 10 min at 55 g (acceleration of 3; slow down at 3; Multifuge 3 S-R, Kendro, Germany). Protoplasts were gently re-suspended in W5 medium (Rother et al. (1994) *J Plant Physiol* 143, 72-77). For counting protoplasts a small volume of the suspension was transferred to a Fuchs-Rosenthal-chamber.

### Transformation protocol

For transformation protoplasts were incubated on ice in the dark for 30 minutes. Subsequently, protoplasts were sedimented by centrifugation at RT for 10 min at 55 g (acceleration of 3; slow down at 3; Multifuge 3 S-R, Kendro). Protoplasts were resuspended in 3M medium (15 mM CaCl₂ x 2H₂O; 0.1 % MES; 0.48 M mannitol; pH 5.6; 540 mOsm; sterile filtered, Schaefer et al. (1991) *Mol Gen Genet* 226, 418-424) at a concentration of 1.2 x 10⁶ protoplasts / ml (Reutter and Reski (1996) Production of a heterologous protein in bioreactor cultures of fully differentiated moss plants, P1. Tissue culture and Biotech., 2, pp. 192-147). 250 microlitre of this protoplast suspension were dispensed into a new sterile centrifuge tube, 50 microlitre DNA solution (column purified DNA in H₂O (Qiagen, Hilden, Germany); 10-100 microlitre; optimal DNA amount of 60 microgram) was added and finally 250 microlitre PEG-solution (40% PEG 4000; 0.4 M mannitol; 0.1 M Ca(NO₃)₂; pH 6 after autoclaving) was added. The suspension was immediately but gently mixed and then incubated for 6 min at RT with occasional gentle mixing. The suspension was diluted progressively by adding 1, 2, 3 and 4 ml of 3M medium. The suspension was centrifuged at 20°C for 10 minutes at 55 g (acceleration of 3; slow down at 3; Multifuge 3 S-R, Kendro). The pellet was re-suspended
a) for transient transformation experiments in 300 microlitre or 400 microlitre 3M medium. Cultivation of transformed protoplasts was performed in 96 well plates (300 microlitre, Nunclon, Nunc GmbH, Wiesbaden , Germany) or 48 well plates (400 microlitre, Cellstar, greiner bio-one, Frickenhausen, Germany).
b) for stable transformation in 3 ml regeneration medium (modified Knop medium; 5% glucose; 3% mannitol; 540 mOsm; pH 5.6-5.8). Regeneration and selection was performed as described by Strepp et al. (1998) *Proc Natl Acad Sci* USA 95,4368-4373). For selection Knop's media were supplemented either with 50 microgram/ml G418 or with 30 microgram/ml Hygromycin B.
Co-transformation was performed by transferring 10 microgram undigested pRT99 (Töpfer et al. (1988) Versatile cloning vectors for transient gene expression and direct gene transfer in plant cells. *NAR* 16, 8725) containing the *npt II* gene as a selection marker in parallel with 10 microgram linearised DNA of each knockout and/or integration construct into the protoplasts.

### MALDI-Tof MS of moss glycans

Plant material was cultivated in liquid culture, isolated by filtration, frozen in liquid nitrogen and stored at -80 °C. Protein bands were excised from Coomassie-stained SDS-polyacrylamide gels. The material was shipped under dry ice. The MALDI-TOF MS analyses were done in the laboratory of Prof. Dr. F. Altmann, Glycobiology Division, Institut für Chemie, Universitat für Bodenkultur, Vienna, Austria.

0.2 to 9 g fresh weight of *Physcomitrella patens* was digested with pepsin. N-glycans were obtained from the digest as described by Wilson et al. (2001). Essentially, the glycans were released by treatment with peptide:N-glycosidase A and analysed by MALDI-TOF mass spectrometry on a DYNAMO (Thermo BioAnalysis, Santa Fe, NM).

### Examples

### 1. Cloning of the 1,2-N-acetylglucosaminyltransferase I from Physcomitrella patens and analysis of the knockout plants

### 1.1 Cloning of the cDNA and genomic DNA for GNTI

A fragment of the cDNA for the GNTI was obtained by performing two rounds of PCR with degenerated primers on the cDNA from P. *patens* WT. These primers were created on the basis of a protein alignment of the known GNTIs from plants. In the first round primers GNT(d)1 (5'-GTNGCNGCNGTNGTNGTNATGGC-3', SEQ ID NO 3) and GTN(d)3 (5'-CCYTTRTANGCNGCNC(TG)NGGNACNCC-3', SEQ ID NO 4) were used and then the product from this PCR was subjected to subsequent PCR with the primers GTN(d)2 (5'-TAYAARATN(CA)GNCAYTAYAARTGG-3', SEQ ID NO 5) and GTN(d)4 (5'-ARRTAYTGYTTRAARAAYTGNCC-3', SEQ ID NO 6). The PCR resulted in a 500bp product which was cloned into pCR 4 TOPO and sequenced from both ends.

To obtain the missing 5'-end of the cDNA 5' RACE was performed with the primers: 5RACEG3 (5'-GTCCGTGTCCAATAAAGGAG-3', SEQ ID NO 7), 5RACEG4 (5'-GTCGGGAGAGATTTCCATGTC-3', SEQ ID NO 8), 5RACEG5 (5'-CTAAGATGACGACCCTTCGG-3', SEQ ID NO 9) which gave an additional 300bp fragment of cDNA, however still without the initial Met. Therefore, on the basis of the new sequence another round of 5' RACE was performed with the primers: 5RACE6 (5'-CATCCTGAGAAACAAAAAGTGG-3', SEQ ID NO 10), 5RACE7 (5'-AGTTACAGACTTCAATGTACG-3', SEQ ID NO 11), and 5RACE8 (5'-AATCAGGACGGTTGCAAGCC-3', SEQ ID NO 12) which gave an additional 400bp fragment containing the initiation codon.

To obtain the missing 3'-end of the cDNA 3' RACE was performed correspondingly with the primers: 3RACEG1 (5'-TTATCCGACCTGAAGTTTGC-3', SEQ ID NO 13), 3RACEG2 (5'-GACCTACAATTTTGGAGAGC-3', SEQ ID NO 14) which resulted in a fragment about 450bp with the stop codon and finally the complete 1416bp of the cDNA for GNTI (GenBank: AJ429143).

The corresponding 5788bp of the genomic sequence was obtained by cloning the GNTI gene in three pieces by PCR on WT *P.patens* genomic DNA with the specific primers: GNT5F (5'-TGGGCTTTAACACAACTTTT-3', SEQ ID NO 15) and GTN6R (5'-GCCCTAAGCTTGATCCCTG-3', SEQ ID NO 16), GNT21F (5'-ATGGCAGATATGGCTCGATTG-3', SEQ ID NO 17) and 5RACEG5 (5'-CTAAGATGACGACCCTTCGG-3', SEQ ID NO 9), 3RACEG1 (5'-TTATCCGACCTGAAGTTTGC-3', SEQ ID NO 13) and GNT15R (5'-AGTTTCTATGGTATCTAACTGC-3', SEQ ID NO 18) which were sequenced by primer walking.

### 1.2 Creating the knockout construct for GNTI

To generate the knockout construct two fragments were synthesized by PCR on the genomic DNA with the primers for the 5'-end of the construct: GNTHT7 (5'-GAGCATCCAAGCTTGACCTGG-3', SEQ ID NO 19) and GNTET7 (5'-GCACCGTGAATTCTTCTAGCTT-3', SEQ ID NO 20) and for the 3'-end correspondingly GNTHT3 (5'-GGAAGAACAAGCTTCAAAGTGGC-3', SEQ ID NO 21) and GNTPT3 (5'-GATCCCTGCAGATCTCAAACG-3', SEQ ID NO 22). The thus obtained fragments of 469bp and 835bp of genomic DNA were first cloned into the TOPO-pCR plasmid (Invitrogen, USA). The fragments were then cut out from this vector with *Eco*RI/*Hind*III and *Pst*I/*Hind*III*,* respectively and cloned into the pCRII vector digested with *Eco*RI and *Pst*I. The resulting plasmid was digested with *Hind*III and the *npt*II selection cassette was introduced, which was obtained by digestion with the same restriction enzyme from the vector pRT101neo (Girke et al. 1998).

For the transformation the knockout plasmid was digested with *Eco*RI and *Bcu*I restriction enzymes and 30microgram were used for the transformation.

### 1.3 Pre-screening of the transgenic plants

For the pre-screening of the resistant plants, small pieces of gametophores (1-5mg) were treated for 30 min at 45°C in 75mM Tris-HCl, pH 8, containing 20 mM (NH₄)₂SO₄ and 0.1% Tween 20, and 3 microlitre of this extract was used for PCR with the following four pairs of primers: GNT7F (5'-GTTCSATGGTTTGAGCAGG-3', SEQ ID NO 23) and GNT8R (5'-GCGACCTTTCCTATTCTCC-3', SEQ ID NO 24) to detect a disruption of the original *gntI* gene, N1 (5'-TACCGACAGTGGTCCCAAAG-3', SEQ ID NO 25) and N2 (5'-CCACCATGATATTCGGCAAG-3', SEQ ID NO 26) to detect the presence of the *npt*II cassette, GNT5F (5'-TGGGCTTTAACACAACTTTT-3', SEQ ID NO 27) and N3 (5'-TGTCGTGCTCCACCATGTT-3', SEQ ID NO 28) to control the integration of the transgene at the 5' end, and N4 (5'-GTTGAGCATATAAGAAAC-3', SEQ ID NO 29) and GNT10R (5'-CACATTGTTCAATTTGATAGAC-3', SEQ ID NO 30) to control the integration at the 3' end. Plants that gave the expected fragments in all four PCR reactions were considered as putative knockouts and selected for further analysis.

Finally 9 transgenic plants were chosen for further molecular and biochemical analysis.

### 1.4 Northern analysis

Total RNA was isolated from moss tissue by RNeasy kit (Qiagen, Germany). For northern analysis, 5 microgram of total RNA were subjected to electrophoresis on formaldehyde-agarose gels, at 120V, transferred to Hybond-N nylon membranes (Amersham Biosciences), and hybridized with a ³²P-labeled cDNA probe corresponding to 3' *gntI* cDNA including part of the homologous region used for the knock out and part of the cDNA not present on the knockout construct. The membranes were washed four times at different concentrations of SSC (the final washing step being 0.5xSSC, 0.1% SDS at 65°C) and exposed to x-ray film (Kodak Bio-Max MS) at -80°C for 2 to 3 days.

Northern analysis with the probe [obtained by PCR on the cDNA with primers: 3RACEG1 (5'-TTATCCGACCTGAAGTTTGC-3', SEQ ID NO 13) and GNT15R (5'-AGTTTCTATGGTATCTAACTGC-3', SEQ ID NO 18)] detected the correct GNTI transcript solely in the wild type. The lack of the correct transcript in the knockout plants corroborates the disruption of the *gntI* gene in *P.patens.*

The expression of the control gene L21 was not affected in the transformants.

### 1.5. Southern analysis of the transgenic plants

For Southern blot analysis 5 microgram of genomic DNA were used. For the detection of the number of integration sites for the *nptII* casette the genomic DNA was digested with PvuII which cuts *nptII* cassette into two fragments and hybridized with the *nptII* cassette as a probe. This analysis showed that some plants have very high numbers of integrated selection cassettes whereas plant No. 6 has a single integration event. Plants No. 4 and 8 have also a low number of integrations: between 4-5 per plant. In a second Southern analysis, genomic DNA was digested with *Eco*RI and hybridized with *gnt*I probe located on the 3' end of the gene, out of the region which was used for creating the knockout construct. In this case it was expected that transgenic plants will have a band which is larger than WT for the size of the *npt*II casette (1500bp). WT have single signal at 4500bp whereas transgenic plants have the bands of 6000bp or other but do not contain the WT band, confirming disruption of the WT locus.

### 1.6 MALDI-TOF mass spectrometry

The N-glycans of *Physcomitrella patens* WT exhibit the typical structural features of plant N-glycans as described in e.g. Wilson et al. (2001) Glycobiology 11, 261-274). I.e. fucose in alpha 1,3-linkage to the Asn-bound GlcNAc, xylose in beta 1,2-linkage to the beta mannosyl reidue, Lewis A epitopes (alpha 1,4-fucosyl and beta 1,3-galactosyl residues linked to GlcNAc) as non reducing terminal elements (tab. 1). Three GNTi knockout plants were analysed. The N-glycans of GNTI knockout plants exhibited the same structures compared to WT (tab. 1) confirming that the knockout was only successful on the molecular but not on the biochemical level. Therefore, it is assumed that another GNTI exists in *Physcomitrella patens.*

### 2. Cloning of the alpha 1,3-fucosyltransferase from P. patens and analysis of the knockout plants

### 2.1. Cloning of the cDNA and genomic DNA for alpha 1,3-fucosyltransferase (alpha 1,3-FT)

Part of the cDNA for the alpha 1,3-FT was obtained by performing two rounds of PCR with degenerated primers on the cDNA from P. *patens* WT. These primers were created on the basis of a protein alignment of the known alpha 1,3-FTs from *Vigna radiata* and *Arabidopsis thaliana.* In the first round primers FD4F (5'-TGGGCNGARTAYGAYATGATG-3', SEQ ID NO 31) and FDR1 (5'-TGNGTNARNCCNADNGGRTADAT-3', SEQ ID NO 32) were used and then the product from this PCR was subjected to a subsequent PCR with the primers FD4F and FD5R (5'-TGNACNGCNGCCATRTC-3', SEQ ID NO 33). The second PCR resulting in a 510bp product which was cloned into pCR 4 TOPO and sequenced from both ends.

To obtain the missing 5'-end of the cDNA, 5' RACE was performed with the primers: 5FT4 (5'-GTAACATTCGCATAATGG-3', SEQ ID NO 34), 5FT5 (5'-CGATCATTATGCGCACCAC-3', SEQ ID NO 35), and 5FT6 (5'-GGAAATAAAAGCAGCTCC-3', SEQ ID NO 36) which gave an additional 200bp of cDNA, however still without the initial Met. Therefore, on the base of the new sequence a second round of 5' RACE was performed with the primers: 5FT7 (5'-AGGGTGAATCTCCATAGCC-3', SEQ ID NO 37), 5FT8 (5'-CATCTGCCTGACCCTCACC-3', SEQ ID NO 38), and 5FT9 (5'-GCCTTGAACACGCATGGC-3', SEQ ID NO 39) which gave an additional 150bp fragment but still without the initiation codon. Finally, a third round was performed with the primers: 5FT9, 5FT10 (5'-CGATACAACCAGCACAGG-3', SEQ ID NO 40), and 5FT11 (5'-CTTCTCTAGCCATTCTGCC-3', SEQ ID NO 41) which gave an additional 400bp of sequence containing the initiation codon.

To obtain missing 3'-end of the cDNA 3' RACE was performed correspondingly with the primers: 3FT1 (5'-GCAGTGGAAGTTTAATGGTC-3', SEQ ID NO 42) and 3FT2 (5'-TCGTTTCTAGCTCTAGTAGAC-3', SEQ ID NO 43) which resulted in a fragment of about 550bp with the stop codon and finally the complete 1711bp of the cDNA for the alpha 1,3-FT (GenBank: partial cDNA: AJ429145).

The corresponding 3083bp of the genomic sequence was obtained by cloning the alpha 1,3-FT gene in three pieces by PCR from WT *P.patens* genomic DNA with the specific primers: FTA9F (5'-ATGCTCCCAGCCCAAGAC-3', SEQ ID NO 44) and FTA10R (5'-TGTCTACTAGAGCTAGAAACG-3', SEQ ID NO 45), FT18F (5'-TAGGGA GTAAATATGAAGGG-3', SEQ ID NO 46) and 5FT5 (5'-CGATCATTATGCGCACCAC-3', SEQ ID NO 35), 3FT1 (5'-GCAGTGGAAGTTTAATGGTC-3', SEQ ID NO 42) and FTA12R (5'-TACTTCCAATTGAAGACAAGG-3', SEQ ID NO 47) which were sequenced by primer walking.

### 2.2. Creating the knockout construct for alpha 1,3-FT

To create the knockout construct, PCR on the genomic DNA was performed with the primers: FT15F (5'-AATGTTCTGTGCCATGCG-3', SEQ ID NO 48) and FT16R (5'-TGCTTCAAATGGGCTAGGG-3', SEQ ID NO 49). The thus obtained 2156bp fragment was cloned into the pCR4 TOPO (Invitrogen, USA) vector. The NptII cassette was synthesized by PCR with a proof reading polymerase on the pRT101neo plasmid (Girke et al. 1998) with the primers: nptII/NdeI-F (5'-ATGCCATATGGCATGCCTGCAGGTCAAC-3', SEQ ID NO 50) to generate a NdeI restriction site and nptII/BstZ17I-R (5'-GCATGTATACGCATGCCTGCAGGTCACTG-3', SEQ ID NO 51) to generate a BstZ17I site. The PCR product was also cloned into pCR4 TOPO (Invitrogen, USA). Both plasmids were digested with NdeI and BstZ17I restriction enzymes. By this restriction a 195bp fragment containing a part of the fourth intron and a part of the fifth exon was cut out from the alpha 1,3-FT genomic fragment and replaced by the *npt*II cassette.

For the transformation of *P. patens* resulting the knockout construct was digested with *Not*I and *Mss*I restriction enzymes and 25 microgram were used for transformation.

### 2.3. Pre-screening of the transgenic plants

For the pre-screening of the resistant plants, small pieces of gametophores (1-5mg) were treated for 30 min at 45°C in 75mM Tris-HCl, pH 8 containing 20 mM (NH₄)₂SO₄ and 0.1% Tween 20, and 3 microlitre of this extract was used for PCR with the following four pairs of primers : FT14F (5'-ACAAAGTTACATACTCGCG-3', SEQ ID NO 52) and FTA12R (5'-TACTTCCAATTGAAGACAAGG-3', SEQ ID NO 47) to detect a disruption of the original ft gene, N1 (5'-TACCGACAGTGGTCCCAAAG-3', SEQ ID NO 25) and N2 (5'-CCACCATGATATTCGGCAAG-3', SEQ ID NO 26) to detect the presence of the *npt*II cassette, FT14F (5'-ACAAAGTTACATACTCGCG-3', SEQ ID NO 52) and N3 (5'-TGTCGTGCTCCACCATGTT-3', SEQ ID NO 28) to control the integration of the transgene at the 5' end, FTA12R (5'-TACTTCCAATTGAAGACAAGG-3', SEQ ID NO 47) and N4 (5'-GTTGAGCATATAAGAAAC-3', SEQ ID NO 29) to control the integration at the 3' end. Plants that gave the expected fragments by all four PCR reactions were considered as putative knockouts and selected for further analysis.

Finally 9 plants were chosen for further molecular and biochemical analysis.

### 2.4. RT-PCR

Total RNA was isolated from moss tissue by RNeasy kit (Qiagen, Germany). Reverse transcription PCR was performed according to standard protocol. For the RT-PCR primers FTA9F (5'-ATGCTCCCAGCCCAAGAC-3', SEQ ID NO 44) and FTA10R (5'-TGTCTACTAGAGCTAGAAACG-3', SEQ ID NO 45) located in the central region of the cDNA for alpha 1,3-FT were used. Using these primers a 475bp transcript was detected only in the WT whereas all transgenic plants did not give any PCR products. The absence of the detectable transcript with the primers located on both sides of integrated nptII cassette confirms that all plants analysed are knockouts.

As a control RT-PCR was performed with primers for APS reductase (Koprivova et al. (2002) J. Biol. Chem. 277, 32195-32201): R10 (5'-TCTTTCACTATTCGGTGACG-3', SEQ ID NO 53) and R11 (5'-CGACCACAACATTAGATCC-3', SEQ ID NO 54), which amplified a 900bp fragment from all plants.

### 2.5 MALDI-TOF mass spectrometry

The N-glycans of *Physcomitrella patens* WT exhibit the typical structural features of plant N-glycans as described in e.g. Wilson et al. (2001), supra. I.E. fucose in alpha 1,3-linkage to the Asn-bound GlcNAc, xylose in beta 1,2-linkage to the beta mannosyl reidue, Lewis A epitopes (alpha 1,4-fucosyl and beta 1,3-galactosyl residues linked to GlcNAc) as non reducing terminal elements (tab. 1). Three alpha 1,3 FT knockout plants were analysed. No alpha 1,3 fucose residues linked to the Asn-bound GlcNAc could be detected on the N-glycans of alpha 1,3 FT knockout plants (tab. 1) confirming that the knockout of alpha 1,3 fucosyltransferase in *Physcomitrella patens* was completely successful.

### 3. Cloning of the beta 1,2-xylosyltransferase from P.patens and analysis of the knockout plants

### 3.1. Cloning of the cDNA and genomic DNA for beta 1,2-xylosyltransferase (beta 1,2 XT)

Part of the cDNA for the beta 1,2-XT was obtained by performing two rounds of PCR with degenerated primers on the cDNA from P. *patens* WT. These primers were created on the basis of a protein alignment of the known beta1,2-XTs from plants. In the first round primers XDF1 (5'-TGYGARGSNTAYTTYGGNAAYGG-3', SEQ ID NO 55) and XDR1 (5'-GCNCKNAYCATYTCNCCRAAYTC-3', SEQ ID NO 56) were used and then the product from this PCR was subjected to a subsequent PCR with the primers XDF2 (5'-GGNGGNGARAARYTNGARRANGT-3', SEQ ID NO 57) and XDR1. The second PCR gave a 610bp product which was cloned into pCR 4 TOPO (Invitrogen, USA) and sequenced from both ends.

To obtain the missing 5'-end of the cDNA, 5' RACE was performed with the primers: 5XT1 (5'-TCCTCCTTCTCTGGGACC-3', SEQ ID NO 58), 5XT2 (5'-AGCTCCAGTTGTGAAATATGG-3', SEQ ID NO 59) and 5XT3 (5'-TTCTTCCTCATTTCGTCCC-3', SEQ ID NO 83) which gave an additional 360bp of cDNA, however still without the initial Met. Therefore, on the basis of the new sequence a second round of 5' RACE was performed with the primers: 5XT4 (5'-CTTCCTTCACCACACTAC-3', SEQ ID NO 60), 5XT5 (5'-TAGCATGACTGTGTGGCC-3', SEQ ID NO 61) and 5XT6 (5'-AAAGGCTTGAGTGTAGCC-3', SEQ ID NO 62) which gave an additional 390bp fragment containing the initiation codon.

To obtain missing 3'-end of the cDNA 3' RACE was performed correspondingly with the primers: 3XT1 (5'-GCCTTTCTTGCACGGGTTG-3', SEQ ID NO 63) and 3XT2 (5'-GGACATTCCAAATAATCCC-3', SEQ ID NO 64) which resulted in a fragment of about 940bp with the stop codon and finally the complete 2300bp of the cDNA for beta 1,2-XT (GenBank: 1788 bp corresponding to the coding region: AJ 429144). The 2388bp of the genomic sequence was obtained by cloning the beta 1,2-XT gene in three pieces by PCR on WT *P*. *patens* genomic DNA with the specific primers: XT14F (5'-TTACGAAGCACACCATGC-3', SEQ ID NO 82) and XT15R (5'-GTCCTGTTAAATGCCTTGC-3', SEQ ID NO 65), XT-M1F (5'-AGGTTGAGCAATCATATGGC-3', SEQ ID NO 66) and 5XT2, 3XT1 and XT11R (5'-ATCCCAGAAATATCTGATCC-3', SEQ ID NO 67) which were sequenced by primer walking.

### 3.2. Creating the knockout construct for beta 1,2-XT

To create the knockout construct PCR on the genomic DNA was performed with the primers: XT12F (5'-TGTGAGGCGTTCTTTGGC-3', SEQ ID NO 68) and XT11R (5'-ATCCCAGAAATATCTGATCC-3', SEQ ID NO 67). The thus obtained fragment of 2066bp fragment was cloned into pCR4 TOPO vector (Invitrogen, USA). The *nptII* cassette was synthesized by PCR on the pRT101neo plasmid (Girke et al. 1998) by proof reading polymerase with the primers: nptII/SalI-F (5'-ATGCGTCGACGTCAACATGGTGGAGCACG-3'. SEQ ID NO 69) for creating *Sal*I restriction site and nptII/Ndel-R (5'-GCATCATATGTCACTGGATTTTGGTTTTAGG-3', SEQ ID NO 70) for creating *Nde*I site. The PCR product was also cloned into pCR4 TOPO (Invitrogen, USA). Both plasmids were digested with *Nde*I and *Sal*I restriction enzymes. From the plasmid with genomic DNA a 380bp fragment containing part of the second exon and part of the second intron was cut out and replaced by the *nptII* cassette was introduced.

For the transformation of P. *patens* the resulting knockout construct was digested with NotI and SpeI restriction enzymes and 25 microgramm were used for transformation.

### 3.3. Pre-screening of the transgenic plants

For the pre-screening of the resistant plants, small pieces of gametophores (1-5mg) were treated for 30 min at 45°C in 75mM Tris-HCl, pH 8 containing 20 mM (NH₄)₂SO₄ and 0.1% Tween 20, and 3 microlitre of this extract was used for PCR with the following four pairs of primers: XT-M1F (5'-AGGTTGAGCAATCATATGGC-3', SEQ ID NO 66) and XT13R (5'-ACGATCCAAAATCTGGACGC-3', SEQ ID NO 71) to detect a disruption of the original xt gene, N1 (5'-TACCGACAGTGGTCCCAAAG-3', SEQ ID NO 25) and N2 (5'-CCACCATGATATTCGGCAAG-3', SEQ ID NO 26) to detect the presence of the *npt*II cassette, XT-M1F (5'-AGGTTGAGCAATCATATGGC-3', SEQ ID NO 66) and N3 (5'-TGTCGTGCTCCACCATGTT-3', SEQ ID NO 28) to control the integration of the transgene at the 5' end, XT13R (5'-ACGATCCAAAATCTGGACGC-3', SEQ ID NO 71) and N4 (5'-GTTGAGCATATAAGAAAC-3', SEQ ID NO 29) and to control the integration at the 3' end. Plants that gave the expected fragments in all four PCR reactions were considered as putative knockouts and selected for further analysis.

Finally 9 plants were chosen for further molecular and biochemical analysis.

### 3.4. RT-PCR

Total RNA was isolated from moss tissue by RNeasy kit (Qiagen, Germany). Reverse transcription PCR was performed according to standard protocol. For the RT-PCR primers XT14F (5'-TTACGAAGCACACCATGC-3', SEQ ID NO 82) and XT15R (5'-GTCCTGTTAAATGCCTGC-3', SEQ ID NO 65) located in the central region of cDNA for beta 1,2-XT and on both sides of *npt II* cassette were used. Using these primers a 290bp transcript was detected only in the WT whereas all transgenic plants did not give any PCR products. The absence of the transcript with the primers located on both sides of the integrated nptII cassette confirms that all plants analysed are knockouts.

As a control RT-PCR was performed with primers for APS reductase (Koprivova et al. 2002), supra): R10 (5'-TCTTTCACTATTCGGTGACG-3', SEQ ID NO 53) and R11 (5'-CGACCACAACATTAGATCC-3', SEQ ID NO 54), which amplified a 900bp fragment from all plants.

### 3.5 MALDI-TOF mass spectrometry

The N-glycans of *Physcomitrella patens* WT exhibit the typical structural features of plant N-glycans as described in e.g. Wilson et al. (2001), supra). I.e. fucose in alpha 1,3-linkage to the Asn-bound GlcNAc, xylose in beta 1,2-linkage to the beta mannosyl reidue, Lewis A epitopes (alpha 1,4-fucosyl and beta 1,3-galactosyl residues linked to GlcNAc) as non reducing terminal elements (tab. 1). Three beta 1,2 XT knockout plants were analysed. No beta 1,2 xylosyl residues linked to the beta-mannosyl residue could be detected on the N-glycans of beta 1,2 XT knockout plants (tab. 1) confirming that the knockout of beta 1,2 xylosyltransferase in *Physcomitrella patens* was completely successful.

### 4. Cloning of the cDNA for human beta-1,4-galactosyltransferase

cDNA from human liver (Invitrogen, USA) was used for isolation of beta 1,4-galactosyltransferase (GalT, GenBank: X55415) cDNA. By using 5' primer GalTXh-F (5'-TTCTCGAGACAATGAGGCTTCGGGAGCCGCTC -3', SEQ ID NO 72) containing a *Xho* I restriction site and 3' primer GalTXb-R (5'-GGTCTAGACTAGCTCGGTGTCCCGATGTCC-3', SEQ ID NO 73) containing a *Xba* I restriction site for PCR with Elongase enzyme mix (Invitrogen, USA) a 1.2 kb DNA fragment was amplified. The 1.2 kb fragment was cloned into pCR4-TOPO (Invitrogen, USA). The fragment was cut out from this vector with *Xho* I and *Xba* I and cloned into pRT101 (Töpfer et al. (1987) *NAR* 15, 5890) digested with *Xho* I and *Xba* I. The resulting plasmid pRT101-GalT contained the cDNA of human beta 1,4-galactosyltransferase under the control of CaMV 35S promoter and CaMV terminator.

### 5. Creating the knockout construct for the beta 1,2-xylosyltransferase and integration of human beta 1,4-galactosyltransferase

From genomic DNA of *Physcomitrella patens* a 1.56 kb fragment of the beta 1,2-xylosyltransferase gene was amplified by using primer XTB-F (5'-TTGGATCCTCAATTACGAAGCACACCATGC-3', SEQ ID NO 74) and primer XTB-R (5'-TTGGATCCTCCTCCCAGAAACATCTGATCCAG-3', SEQ ID NO 75), both primers introduced Bam HI restriction sites. The amplification product was cloned into pCR4-TOPO (Invitrogen, USA). The cloned beta 1,2-xylosyltransferase gene fragment contained a single Hind III restriction site. In this Hind III restriction site the cDNA of beta 1,4-galactosyltransferase under the control of CaMV 35S promoter and CaMV terminator was introduced by ligation, resulting in the plasmid pCR4-XTko-GalTki. Digestion of pCR4-XTko-GalTki with Bam HI resulted in a fragment which contained the cDNA of beta 1,4-galactosyltransferase under the control of CaMV 35S promoter and CaMV terminator flanked 5' and 3' by sequences homolog to the beta 1,2-xylosyltransferase gene of *Physcomitrella patens.* This fragment was used for knock out experiments.

### 5.1 PCR

For PCR analyses genomic DNA isolated from the putative knockout plants was used. The primers MoB 521 (5'-TTGCCGCTATCTACTTGTATGCTAACGT-3', SEQ ID NO 76) and MoB 575 (5'-TGCCGTGGATGTGCTAGATAATCTT-3', SEQ ID NO 77) were located on the sequences homologous to the beta 1,2-xylosyltransferase on both sides of the beta 1,4-galactosyltransferase cassette. A fragment of 339 bp corresponding to the expected beta 1,2-xylosyltransferase sequence was amplified from WT. From knockout plants a 2.1 kbp fragment corresponding to the introduced beta 1,4-galactosyltransferase cassette was amplified and no 339 bp fragment could be detected, confirming the knockout of beta 1,2-xylosyltransferase as well as the integration of the beta 1,4-galactosyltransferase cassette.

### 5.2 MALDI-TOF mass spectrometry

The N-glycans of *Physcomitrella patens* WT exhibit the typical structural features of plant N-glycans as described in e.g. Wilson et al. (2001), supra. I.e. fucose in alpha 1,3-linkage to the Asn-bound GlcNAc, xylose in beta 1,2-linkage to the beta mannosyl reidue, Lewis A epitopes (alpha 1,4-fucosyl and beta 1,3-galactosyl residues linked to GlcNAc) as non reducing terminal elements (tab. 1). Three beta 1,2 XT knockout, beta 1,4 GT integration plants were analysed. No beta 1,2 xylosyl residues linked to the beta-mannosyl residue could be detected on the N-glycans of these plants. The peak at 2235 found in WT describing the (GF)(GF)XF structure was shifted in the N-glycan pattern of the transgenic plants confirming the activity of the human beta 1,4 galactosyltransferase in *Physcomitrella patens.*

### 6. Creating the knockout construct for the alpha 1,3-fucosyltransferase and integration of human beta 1,4-galactosyltransferase

From genomic DNA of *Physcomitrella patens* a 2.66 kb fragment of the alpha 1,3-fucosyltransferase gene was amplified by using primer FTB-F (5'-TAGGATCCAGATGATGTCTGCTCGGCAGAATGG-3', SEQ ID NO 78) and primer FTB-R (5'-CTGGATCCTTGTAGATCCGAAGGTCTGAGTTCC-3', SEQ ID NO 79), both primers introduced *Bam* HI restriction sites. The amplification product was cloned into pCR4-TOPO (Invitrogen, USA). The cloned alpha 1,3-fucosyltransferase gene fragment contained two Hind III restriction sites. In these Hind III restriction sites the cDNA of beta 1,4-galactosyltransferase under the control of CaMV 35S promoter and CaMV terminator was introduced by ligation, resulting in the plasmid pCR4-FTko-GalTki. Digestion of pCR4-FTko-GalTki with *Bam* HI resulted in a fragment which contained the cDNA of beta 1,4-galactosyltransferase under the control of CaMV 35S promoter and CaMV terminator flanked 5' and 3' by sequences homolog to the beta 1,3-fucosyltransferase gene of *Physcomitrella patens.* This fragment was used for knock out experiments.

### 6.1 PCR

For PCR analyses genomic DNA isolated from the putative knockout plants was used. The primers MoB 435 (5'-TCCTACCTGCGGAGCAACAGATATTG-3', SEQ ID NO 80) and MoB 495 (5'-GTGGACCCAGATTTGCTGGTGCACTTG-3', SEQ ID NO 81) were located on the sequences homologous to the alpha 1,3-fucosyltransferase on both sides of the beta 1,4-galactosyltransferase cassette. A fragment of 2 kbp corresponding to the expected alpha 1,3-fucosyltransferase sequence was amplified from WT. From knockout plants a 2.8 kbp fragment corresponding to the introduced beta 1,4-galactosyltransferase cassette was amplified and no 2 kbp fragment could be detected, confirming the knockout of alpha 1,3-fucosyltransferase as well as the integration of the beta 1,4-galactosyltransferase cassette.

### 6.2 MALDI-TOF mass spectrometry

The N-glycans of *Physcomitrella patens* WT exhibit the typical structural features of plant N-glycans as described in e.g. Wilson et al. (2001), supra. I.e. fucose in alpha 1,3-linkage to the Asn-bound GlcNAc, xylose in beta 1,2-linkage to the beta mannosyl reidue, Lewis A epitopes (alpha 1,4-fucosyl and beta 1,3-galactosyl residues linked to GlcNAc) as non reducing terminal elements (tab. 1). Three alpha 1,3 FT knockout, beta 1,4 GT integration plants were analysed. No alpha 1,3 fucosyl residues linked to the Asn-bound GlcNAc could be detected on the N-glycans of these plants. The peak at 2235 found in WT describing the (GF)(GF)XF structure was shifted in the N-glycan pattern of the transgenic plants confirming the activity of the human beta 1,4 galactosyltransferase in *Physcomitrella patens.*

### 7. Generating plants with alpha 1,3 fucosyltransferase and beta 1,2 xylosyltransferase knockouts and beta 1,4-galactosyltransferase integration

For generating plants without alpha 1,3-fucosyl and beta 1,2-xylosyl residues and with beta 1,4 linked galactosyl residues protoplasts derived of *Physcomitrella patens* protonema were transformed with the constructs described in chapter 5 and 6. Transformation could be performed subsequently as well as co-transformation with both constructs.

### 7.1 PCR

For PCR analyses same procedures as described in 5.1 and 6.1 were performed.

### 7.2 MALDI-TOF mass spectrometry

The N-glycans of *Physcomitrella patens* WT exhibit the typical structural features of plant N-glycans as described in e.g. Wilson et al. (2001), supra. I.e. fucose in alpha 1,3-linkage to the Asn-bound GlcNAc, xylose in beta 1,2-linkage to the beta mannosyl reidue, Lewis A epitopes (alpha 1,4-fucosyl and beta 1,3-galactosyl residues linked to GlcNAc) as non reducing terminal elements (tab. 1). Three transgenic plants were analysed. No alpha 1,3 fucosyl residues linked to the Asn-bound GlcNAc nor beta 1,2-xylosyl residues could be detected on the N-glycans of these plants. The peak at 2235 found in WT describing the (GF)(GF)XF structure was shifted to mass peak of 1665 in the N-glycan pattern of the transgenic plants confirming the activity of the human beta 1,4 galactosyltransferase in *Physcomitrella patens* as well as the loss of 1,3 linked fucosyl and 1,2 linked xylosyl residues.

### 8.1 Purification and analysis of recombinant human VEGF₁₂₁ expressed in transiently transformed Physcomitrella protoplasts

Protoplasts derived from protonema of tansgenic Physcomitrella plants containing human beta 1,4 galactosyltransferasen and no alpha 1,3 fucoslytransferase nor beta 1,2 xylosyltransferase (7.2) were transformed with pRT101TPVEGF C3. Expressed VEGF₁₂₁ was secreted into the medium. After two, three and four days the culture medium was collected and replaced by fresh medium. Samples were filtered through a 0.22 microm Millex GP filter unit (Millipore). Recombinant VEGF₁₂₁ was purified by FPLC (Aktaexplorer 100, Amersham Biosciences, Germany) using a SP-Sepharose column (Amersham Biosciences, Germany). Elution was performed with sodium chloride in 25 mM Na-acetate, pH 5.0. Eluted fractions were concentrated by centrifugation using Amicon Ultra filter devices, cut off 10.000 (Millipore).

SDS/PAGE loading buffer was added to the samples, proteins were fractionated on SDS/PAGE containing 15 % acrylamide and 0.4 % bisacrylamide. Gels were stained with Coomassie Brilliant Blue R-250. VEGF121 band was excised from Coomassie stained SDS/PAGE and digested with sequencing grade trypsin. The glycans were released by treatment with peptide:N-glycosidase A and analysed by MALDI-TOF mass spectrometry on a DYNAMO (Thermo BioAnalysis, Santa Fe, NM).

Detected N-glycan pattern of secreted recombinant VEGF₁₂₁ was similar to that observed in transgenic GalT (with knockouts of FucT and XylT, see 7.2) plants confirming the activity of the human beta 1,4 galactosyltransferase in *Physcomitrella patens* as well as the loss of 1,3 linked fucosyl and 1,2 linked xylosyl residues.

**Tab.1: N-glycan structures of Physcomitrella patens wildtype (WT)and knock out plants. N-glycans were isolated from plant material grown under same conditions (500 ml flasks, modified Knop medium). F = fucosyl residue, G = galactosyl residue, Gn = N-acetylglucosaminyl residue, M / Man = mannosyl residue, X = xylosyl residue. (GF)(GF)XF depicts the most complex type N-glycan, the so called Lewis a formation.**

| | WT | GNT1 Ko | FucT Ko | XylT Ko |
|---|---|---|---|---|
| M+ Na | Structures normal | Structures normal | Structures without alpha 1,3- fucosyl residues | Structures without alpha 1,3- xylosyl residues |
| | | | | |
| 933.8 | Man3 (MM) | Man3 (MM) | Man3 (MM) | Man3 (MM) |
| 1065,7 | MMX | MMX | MMX | |
| 1080,0 | MMF | MMF | | MMF |
| 1096,0 | Man4 | Man4 | Man4 | Man4 |
| 1137.0 | MGn /GnM | MGn /GnM | MGn /GnM | MGn /GnM |
| 1212,1 | MMXF | MMXF | | |
| 1227,8 | Man4X | Man4X | | |
| 1258,4 | Man5 | Man5 | Man5 | Man5 |
| 1269,1 | GnMX/MGnX | GnMX/MGnX | GnMX/MGnX | |
| 1283,4 | | | | GnMF/MGnF |
| 1299,2 | Man4Gn | Man4Gn | Man4Gn | Man4Gn |
| 1340,2 | GnGn | GnGn | GnGn | GnGn |
| 1415,5 | GnMXF/MGnXF | GnMXF/MGnXF | | |
| 1420,2 | Man6 | Man6 | Man6 | Man6 |
| 1431,4 | Man4G nX | Man4GnX | M an4G nX | |
| 1445,3 | Man4GnF | Man4GnF | | Man4GnF |
| 1472,1 | GnGnX | GnGnX | GnGnX | |
| 1486,4 | GnGnF | GnGnF | | GnGnF |
| 1577,4 | GMXF/MGXP/ Man4GnXF | GMXF/MGXF/ Man4GnXF | | |
| 1582,4 | Man7 | Man7 | Man7 | Man7 |
| 1618.5 | GnGnXF | G nG nXF | | |
| 1739,5 | Man5GnXF | Man5GnXF | | |
| 1744,5 | Man8 | Man8 | Man8 | Man8 |
| 1780,4 | | | (GF)GnX / G n(G F)X | |
| 1794,9 | | | | (GF)GnF / Gn(GF)F |
| 1907,1 | Man9 | Man9 | Man9 | Man9 |
| 1926.7 | (GF)GnXF/Gn(GF)XF | (GF)GnXF/Gn(GF)XF | | |
| 2068,8 | Man9Glc1 | Man9Glc1 | | |
| 2088,9 | | | (GF)(GF)X | |
| 2102,8 | | | | (G F)(G F)F |
| 2235,0 | (GF)(GF)XF | (GF)(GF)XF | | |

### SEQUENCE LISTING

<110> greenovation Biotech GmbH
<120> Improvements in or relating to protein production
<130> Protein production
<140> 02028536.7
   <141> 2002-12-20
<160> 83
<170> Patent In Ver. 2.1
<210> 1
   <211> 36
   <212> DNA
   <213> Artificial Sequence
<220>
   <223> Description of Artificial Sequence: Primer sequence MoB323
<400> 1
   atactcgagg aagatgaact tttctgcctg tcttgg
   36
<210> 2
   <211> 26
   <212> DNA
   <213> Artificial Sequence
<220>
   <223> Description of Artificial Sequence: Primer sequence MoB349
<400> 2
   ctgccatggg tgcagcctgg gaccac
   26
<210> 3
   <211> 23
   <212> DNA
   <213> Artificial Sequence
<220>
   <223> Description of Artificial Sequence: Primer sequence GNT(d)1
<400> 3
   gtngcngcng tngtngtnat ggc
   23
<210> 4
   <211> 27
   <212> DNA
   <213> Artificial Sequence
<220>
   <223> Description of Artificial Sequence: Primer sequence GTN(d)3
<400> 4
   ccyttrtang cngcnctgng gnacncc
   27
<210> 5
   <211> 25
   <212> DNA
   <213> Artificial Sequence
<220>
   <223> Description of Artificial Sequence: Primer sequence GTN (d) 2
<400> 5
   tayaaratnc agncaytaya artgg
   25
<210> 6
   <211> 23
   <212> DNA
   <213> Artificial Sequence
<220>
   <223> Description of Artificial Sequence: Primer sequence GTN(d)4
<400> 6
   arrtaytgyt traaraaytg ncc
   23
<210> 7
   <211> 20
   <212> DNA
   <213> Artificial Sequence
<220>
   <223> Description of Artificial Sequence: Primer sequence 5RACEG3
<400> 7
   gtccgtgtcc aataaaggag
   20
<210> 8
   <211> 21
   <212> DNA
   <213> Artificial Sequence
<220>
   <223> Description of Artificial Sequence: Primer sequence 5RACEG4
<400> 8
   gtcgggagag atttccatgt c
   21
<210> 9
   <211> 20
   <212> DNA
   <213> Artificial Sequence
<220>
   <223> Description of Artificial Sequence: Primer sequence 5RACEG5
<400> 9
   ctaagatgac gacccttcgg
   20
<210> 10
   <211> 22
   <212> DNA
   <213> Artificial Sequence
<220>
   <223> Description of Artificial Sequence: Primer sequence 5RACE6
<400> 10
   catcctgaga aacaaaaagt gg
   22
<210> 11
   <211> 21
   <212> DNA
   <213> Artificial Sequence
<220>
   <223> Description of Artificial Sequence: Primer sequence 5RACE7
<400> 11
   agttacagac ttcaatgtac g
   21 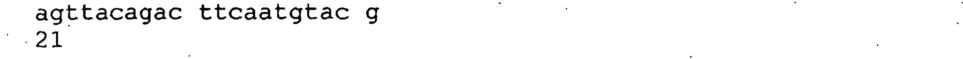
<210> 12
   <211> 20
   <212> DNA
   <213> Artificial Sequence
<220>
   <223> Description of Artificial Sequence: Primer sequence 5RACE8
<400> 12
   aatcaggacg gttgcaagcc
   20 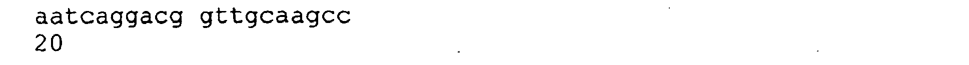
<210> 13
   <211> 20
   <212> DNA
   <213> Artificial Sequence
<220>
   <223> Description of Artificial Sequence: Primer sequence 3RACEG1
<400> 13
   ttatccgacc tgaagtttgc
   20 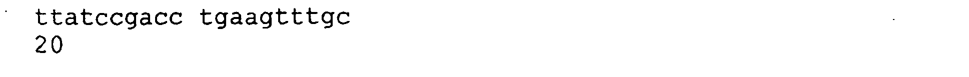
<210> 14
   <211> 20
   <212> DNA
   <213> Artificial Sequence
<220>
   <223> Description of Artificial Sequence: Primer sequence 3RACEG2
<400> 14
   gacctacaat tttggagagc
   20
<210> 15
   <211> 20
   <212> DNA
   <213> Artificial Sequence
<220>
   <223> Description of Artificial Sequence: Primer sequence GNT5F
<400> 15
   tgggctttaa cacaactttt
   20
<210> 16
   <211> 19
   <212> DNA
   <213> Artificial Sequence
<220>
   <223> Description of Artificial Sequence: Primer sequence GTN6R
<400> 16
   gccctaagct tgatccctg
   19
<210> 17
   <211> 21
   <212> DNA
   <213> Artificial Sequence
<220>
   <223> Description of Artificial Sequence: Primer sequence GNT21F
<400> 17
   atggcagata tggctcgatt g
   21
<210> 18
   <211> 22
   <212> DNA
   <213> Artificial Sequence
<220>
   <223> Description of Artificial Sequence: Primer sequence GNT15R
<400> 18
   agtttctatg gtatctaact gc
   22
<210> 19
   <211> 21
   <212> DNA
   <213> Artificial Sequence
<220>
   <223> Description of Artificial Sequence: Primer sequence GNTHT7
<400> 19
   gagcatccaa gcttgacctg g
   21
<210> 20
   <211> 22
   <212> DNA
   <213> Artificial Sequence
<220>
   <223> Description of Artificial Sequence: Primer sequence GNTET7
<400> 20
   gcaccgtgaa ttcttctagc tt
   22
<210> 21
   <211> 23
   <212> DNA
   <213> Artificial Sequence
<220>
   <223> Description of Artificial Sequence: 1 sequence GNTHT3
<400> 21
   ggaagaacaa gcttcaaagt ggc
   23
<210> 22
   <211> 21
   <212> DNA
   <213> Artificial Sequence
<220>
   <223> Description of Artificial Sequence: Primer sequence GNTPT3.
<400> 22
<210> 23
   <211> 19
   <212> DNA
   <213> Artificial Sequence
<220>
   <223> Description of Artificial Sequence: Primer sequence GNT7F
<400> 23
   gttcsatggt ttgagcagg
   19
<210> 24
   <211> 19
   <212> DNA
   <213> Artificial Sequence
<220>
   <223> Description of Artificial Sequence: Primer sequence GNT8R
<400> 24
   gcgacctttc ctattctcc
   19
<210> 25
   <211> 20
   <212> DNA
   <213> Artificial Sequence
<220>
   <223> Description of Artificial Sequence: Primer sequence N1
<400> 25
   taccgacagt ggtcccaaag
   20
<210> 26
   <211> 20
   <212> DNA
   <213> Artificial Sequence
<220>
   <223> Description of Artificial Sequence: Primer sequence N2
<400> 26
   ccaccatgat attcggcaag
   20
<210> 27
   <211> 20
   <212> DNA
   <213> Artificial Sequence
<220>
   <223> Description of Artificial Sequence: Primer sequence GNT5F
<400> 27
   tgggctttaa cacaactttt
   20
<210> 28
   <211> 19
   <212> DNA
   <213> Artificial Sequence
<220>
   <223> Description of Artificial Sequence: Primer sequence N3
<400> 28
   tgtcgtgctc caccatgtt
   19
<210> 29
   <211> 18
   <212> DNA
   <213> Artificial Sequence
<220>
   <223> Description of Artificial Sequence: Primer sequence N4
<400> 29
   gttgagcata taagaaac
   18
<210> 30
   <211> 22
   <212> DNA
   <213> Artificial Sequence
<220>
   <223> Description of Artificial Sequence: Primer sequence GNT10R
<400> 30
   cacattgttc aatttgatag ac
   22
<210> 31
   <211> 21
   <212> DNA
   <213> Artificial Sequence
<220>
   <223> Description of Artificial Sequence: Primer sequence FD4F
<400> 31
   tgggcngart aygayatgat g
   21
<210> 32
   <211> 23
   <212> DNA
   <213> Artificial Sequence
<220>
   <223> Description of Artificial Sequence: Primer sequence FDR1
<400> 32
   tgngtnarnc cnadnggrta dat
   23
<210> 33
   <211> 17
   <212> DNA
   <213> Artificial Sequence
<220>
   <223> Description of Artificial Sequence: Primer sequence FD5R
<400> 33
   tgnacngcng ccatrtc
   17
<210> 34
   <211> 18
   <212> DNA
   <213> Artificial Sequence
<220>
   <223> Description of Artificial Sequence: Primer sequence 5FT4
<400> 34
   gtaacattcg cataatgg
   18
<210> 35
   <211> 19
   <212> DNA
   <213> Artificial Sequence
<220>
   <223> Description of Artificial Sequence: Primer sequence 5FT5
<400> 35
   cgatcattat gcgcaccac
   19
<210> 36
   <211> 18
   <212> DNA
   <213> Artificial Sequence
<220>
   <223> Description of Artificial Sequence: Primer sequence 5FT6
<400> 36
   ggaaataaaa gcagctcc
   18
<210> 37
   <211> 19
   <212> DNA
   <213> Artificial Sequence
<220>
   <223> Description of Artificial Sequence: Primer sequence 5FT7
<400> 37
   agggtgaatc tccatagcc
   19
<210> 38
   <211> 19
   <212> DNA
   <213> Artificial Sequence
<220>
   <223> Description of Artificial Sequence: Primer sequence 5FT8
<400> 38
   catctgcctg accctcacc
   19
<210> 39
   <211> 18
   <212> DNA
   <213> Artificial Sequence
<220>
   <223> Description of Artificial Sequence: Primer sequence 5FT9
<400> 39
   gccttgaaca cgcatggc
   18
<210> 40
   <211> 18
   <212> DNA
   <213> Artificial Sequence
<220>
   <223> Description of Artificial Sequence: Primer sequence 5FT10
<400> 40
   cgatacaacc agcacagg
   18
<210> 41
   <211> 19
   <212> DNA
   <213> Artificial Sequence
<220>
   <223> Description of Artificial Sequence: Primer sequence 5FT11
<400> 41
   cttctctagc cattctgcc
   19
<210> 42
   <211> 20
   <212> DNA
   <213> Artificial Sequence
<220>
   <223> Description of Artificial Sequence: Primer sequence 3FT1
<400> 42
   gcagtggaag tttaatggtc
   20
<210> 43
   <211> 21
   <212> DNA
   <213> Artificial Sequence
<220>
   <223> Description of Artificial Sequence: Primer sequence 3FT2
<400> 43
   tcgtttctag ctctagtaga c
   21
<210> 44
   <211> 18
   <212> DNA
   <213> Artificial Sequence
<220>
   <223> Description of Artificial Sequence: Primer sequence FTA9F
<400> 44
   atgctcccag cccaagac
   18
<210> 45
   <211> 21
   <212> DNA
   <213> Artificial Sequence
<220>
   <223> Description of Artificial Sequence: Primer sequence FTA10R
<400> 45
   tgtctactag agctagaaac g
   21
<210> 46
   <211> 20
   <212> DNA
   <213> Artificial Sequence
<220>
   <223> Description of Artificial Sequence: Primer sequence FT18F
<400> 46
   tagggagtaa atatgaaggg
   20
<210> 47
   <211> 21
   <212> DNA
   <213> Artificial Sequence
<220>
   <223> Description of Artificial Sequence: Primer sequence FTA12R
<400> 47
   tacttccaat tgaagacaag g
   21
<210> 48
   <211> 18
   <212> DNA
   <213> Artificial Sequence
<220>
   <223> Description of Artificial Sequence: Primer sequence FT15F
<400> 48
   aatgttctgt gccatgcg
   18
<210> 49
   <211> 19
   <212> DNA
   <213> Artificial Sequence
<220>
   <223> Description of Artificial Sequence: Primer sequence FT16R
<400> 49
   tgcttcaaat gggctaggg
   19
<210> 50
   <211> 28
   <212> DNA
   <213> Artificial Sequence
<220>
   <223> Description of Artificial Sequence: Primer sequence nptII/NdeI-F
<400> 50
   atgccatatg gcatgcctgc aggtcaac
   28
<210> 51
   <211> 29
   <212> DNA
   <213> Artificial Sequence
<220>
   <223> Description of Artificial Sequence: Primer sequence nptII/BstZ17I-R
<400> 51
   gcatgtatac gcatgcctgc aggtcactg
   29
<210> 52
   <211> 19
   <212> DNA
   <213> Artificial Sequence
<220>
   <223> Description of Artificial Sequence: Primer sequence FT14F
<400> 52
   acaaagttac atactcgcg
   19
<210> 53
   <211> 20
   <212> DNA
   <213> Artificial Sequence
<220>
   <223> Description of Artificial Sequence: Primer sequence R10
<400> 53
   tctttcacta ttcggtgacg
   20
<210> 59
   <211> 19
   <212> DNA
   <213> Artificial Sequence
<220>
   <223> Description of Artificial Sequence: Primer sequence R11
<400> 54
<210> 55
   <211> 23
   <2.12> DNA
   <213> Artificial Sequence
<220>
   <223> Description of Artificial Sequence: Primer sequence XDF1
<400> 55
   tgygargsnt ayttyggnaa ygg
   23
<210> 56
   <211> 23
   <212> DNA
   <213> Artificial Sequence
<220>
   <223> Description of Artificial Sequence: Primer sequence XDR1
<400> 56
   gcncknayca tytcnccraa ytc
   23
<210> 57
   <211> 23
   <212> DNA
   <213> Artificial Sequence
<220>
   <223> Description of Artificial Sequence: Primer sequence XDF2
<400> 57
   ggnggngara arytngarra ngt
   23
<210> 58
   <211> 18
   <212> DNA
   <213> Artificial Sequence
<220>
   <223> Description of Artificial Sequence: Primer sequence 5XT1
<400> 58
   tcctccttct ctgggacc
   18
<210> 59
   <211> 21
   <212> DNA
   <213> Artificial Sequence
<220>
   <223> Description of Artificial Sequence: Primer sequence 5XT2
<400> 59
   agctccagtt gtgaaatatg g
   21
<210> 60
   <211> 18
   <212> DNA
   <213> Artificial Sequence
<220>
   <223> Description of Artificial Sequence: Primer sequence 5XT4
<400> 60
   cttccttcac cacactac
   18
<210> 61
   <211> 18
   <212> DNA
   <213> Artificial Sequence
<220>
   <223> Description of Artificial Sequence: Primer sequence 5XT5
<400> 61
   tagcatgact gtgtggcc
   18
<210> 62
   <211> 18
   <212> DNA
   <213> Artificial Sequence
<220>
   <223> Description of Artificial Sequence: Primer sequence 5XT6
<400> 62
   aaaggcttga gtgtagcc
   18
<210> 63
   <211> 19
   <212> DNA
   <213> Artificial Sequence
<220>
   <223> Description of Artificial Sequence: Primer sequence 3XT1
<400> 63
   gcctttcttg cacgggttg
   19
<210> 64
   <211> 19
   <212> DNA
   <213> Artificial Sequence
<220>
   <223> Description of Artificial Sequence: Primer sequence 3XT2
<400> 64
   ggacattcca aataatccc
   19
<210> 65
   <211> 19
   <212> DNA
   <213> Artificial Sequence
<220>
   <223> Description of Artificial Sequence: Primer sequence XT15R
<400> 65
   gtcctgttaa atgccttgc
   19
<210> 66
   <211> 20
   <212> DNA
   <213> Artificial Sequence
<220>
   <223> Description of Artificial Sequence: Primer sequence XT-M1F
<400> 66
   aggttgagca atcatatggc
   20
<210> 67
   <211> 20
   <212> DNA
   <213> Artificial Sequence
<220>
   <223> Description of Artificial Sequence: Primer sequence XT11R
<400> 67
   atcccagaaa tatctgatcc
   20
<210> 68
   <211> 18
   <212> DNA
   <213> Artificial Sequence
<220>
   <223> Description of Artificial Sequence: Primer sequence XT12F
<400> 68
   tgtgaggcgt tctttggc
   18
<210> 69
   <211> 29
   <212> DNA
   <213> Artificial Sequence
<220>
   <223> Description of Artificial Sequence: Primer sequence nptII/SalI-F
<400> 69
   atgcgtcgac gtcaacatgg tggagcacg
   29
<210> 70
   <211> 31
   <212> DNA
   <213> Artificial Sequence
<220>
   <223> Description of Artificial Sequence: Primer sequence nptII/NdeI-R
<400> 70
   gcatcatatg tcactggatt ttggttttag g
   31
<210> 71
   <211> 20
   <212> DNA
   <213> Artificial Sequence
<220>
   <223> Description of Artificial Sequence: Primer sequence XT13R
<400> 71
   acgatccaaa atctggacgc
   20
<210> 72
   <211> 32
   <212> DNA.
   <213> Artificial Sequence
<220>
   <223> Description of Artificial Sequence: Primer sequence GalTXh-F
<400> 72
   ttctcgagac aatgaggctt cgggagccgc tc
   32
<210> 73
   <211> 30
   <212> DNA
   <213> Artificial Sequence
<220>
   <223> Description of Artificial Sequence: Primer sequence GalTXb-R
<400> 73
   ggtctagact agctcggtgt cccgatgtcc
   30
<210> 74
   <211> 30
   <212> DNA
   <213> Artificial Sequence
<220>
   <223> Description of Artificial Sequence: Primer sequence XTB-F
<400> 74
   ttggatcctc aattacgaag cacaccatgc
   30
<210> 75
   <211> 32
   <212> DNA
   <213> Artificial Sequence
<220>
   <223> Description of Artificial Sequence: Primer sequence XTB-R
<400> 75
   ttggatcctc ctcccagaaa catctgatcc ag
   32
<210> 76
   <211> 28
   <212> DNA
   <213> Artificial Sequence
<220>
   <223> Description of Artificial Sequence: Primer sequence MoB521
<400> 76
   ttgccgctat ctacttgtat gctaacgt
   28
<210> 77
   <211> 25
   <212> DNA
   <213> Artificial Sequence
<220>
   <223> Description of Artificial Sequence: Primer sequence MoB575
<400> 77
   tgccgtggat gtgctagata atctt
   25
<210> 78
   <211> 33
   <212> DNA
   <213> Artificial Sequence
<220>
   <223> Description of Artificial Sequence: Primer sequence FTB-F
<400> 78
   taggatccag atgatgtctg ctcggcagaa tgg
   33
<210> 79
   <211> 33
   <212> DNA
   <213> Artificial Sequence
<220>
   <223> Description of Artificial Sequence: Primer sequence FTB-R
<400> 79
   ctggatcctt gtagatccga aggtctgagt tcc
   33
<210> 80
   <211> 26
   <212> DNA
   <213> Artificial Sequence
<220>
   <223> Description of Artificial Sequence: Primer sequence MoB435
<400> 80
   tcctacctgc ggagcaacag atattg
   26
<210> 81
   <211> 27
   <212> DNA
   <213> Artificial Sequence
<220>
   <223> Description of Artificial Sequence: Primer sequence MoB495
<400> 81
   gtggacccag atttgctggt gcacttg
   27
<210> 82
   <211> 18
   <212> DNA
   <213> Artificial Sequence
<220>
   <223> Description of Artificial Sequence: Primer sequence XT14F
<400> 82
   ttacgaagca caccatgc
   18
<210> 83
   <211> 19
   <212> DNA
   <213> Artificial Sequence
<220>
   <223> Description of Artificial Sequence: Primer sequence 5XT3
<400> 83
   ttcttcctca tttcgtccc
   19

### SEQUENCE LISTING

<110> greenovation Biotech GmbH
<120> Improvements in or relating to protein production
<130> Protein production
<140> 02028536.7
   <141> 2002-12-20
<160> 83
<170> PatentIn Ver. 2.1
<210> 1
   <211> 36
   <212> DNA
   <213> Artificial Sequence
<220>
   <223> Description of Artificial Sequence: Primer sequence MoB323
<400> 1
   atactcgagg aagatgaact tttctgcctg tcttgg
   36
<210> 2
   <211> 26
   <212> DNA
   <213> Artificial Sequence
<220>
   <223> Description of Artificial Sequence: Primer sequence MoB349
<400> 2
   ctgccatggg tgcagcctgg gaccac
   26
<210> 3
   <211> 23
   <212> DNA
   <213> Artificial Sequence
<220>
   <223> Description of Artificial Sequence: Primer sequence GNT (d) 1
<400> 3
   gtngcngcng tngtngtnat ggc
   23
<210> 4
   <211> 27
   <212> DNA
   <213> Artificial Sequence
<220>
   <223> Description of Artificial Sequence: Primer sequence GTN(d)3
<400> 4
   ccyttrtang cngcnctgng gnacncc
   27
<210> 5
   <211> 25
   <212> DNA
   <213> Artificial Sequence
<220>
   <223> Description of Artificial Sequence: Primer sequence GTN(d)2
<400> 5
   tayaaratnc agncaytaya artgg
   25
<210> 6
   <211> 23
   <212> DNA
   <213> Artificial Sequence
<220>
   <223> Description of Artificial Sequence: Primer sequence GTN(d)4 4
<400> 6
   arrtaytgyt traaraaytg ncc
   23
<210> 7
   <211> 20
   <212> DNA
   <213> Artificial Sequence
<220> Primer
   <223> Description of Artificial Sequence: Primer sequence 5RACEG3
<400> 7
   gtccgtgtcc aataaaggag
   20
<210> 8
   <211> 21
   <212> DNA
   <213> Artificial Sequence
<220>
   <223> Description of Artificial Sequence: Primer sequence 5RACEG4
<400> 8
   gtcgggagag atttccatgt c
   21
<210> 9
   <211> 20
   <212> DNA
   <213> Artificial Sequence
<220>
   <223> Description of Artificial Sequence: Primer sequence 5RACEG5
<400> 9
   ctaagatgac gacccttcgg
   20
<210> 10
   <211> 22
   <212> DNA
   <213> Artificial Sequence
<220>
   <223> Description of Artificial Sequence: Primer sequence 5RACE6
<400> 10
   catcctgaga aacaaaaagt gg
   22
<210> 11
   <211> 21
   <212> DNA
   <213> Artificial Sequence
<220>
   <223> Description of Artificial Sequence: Primer sequence 5RACE7
<400> 11
   agttacagac ttcaatgtac g
   21
<210> 12
   <211> 20
   <212> DNA
   <213> Artificial Sequence
<220>
   <223> Description of Artificial Sequence: Primer sequence 5RACE8
<400> 12
   aatcaggacg gttgcaagcc
   20
<210> 13
   <211> 20
   <212> DNA
   <213> Artificial Sequence
<220>
   <223> Description of Artificial Sequence: Primer sequence 3RACEG1
<400> 13
   ttatccgacc tgaagtttgc
   20
<210> 14
   <211> 20
   <212> DNA
   <213> Artificial Sequence
<220>
   <223> Description of Artificial Sequence: Primer sequence 3RACEG2
<400> 14
   gacctacaat tttggagagc
   20
<210> 15
   <211> 20
   <212> DNA
   <213> Artificial Sequence
<220>
   <223> Description of Artificial Sequence: Primer sequence GNT5F
<400> 15
   tgggctttaa cacaactttt
   20
<210> 16
   <211> 19
   <212> DNA
   <213> Artificial Sequence
<220>
   <223> Description of Artificial Sequence: Primer sequence GTN6R
<400> 16
   gccctaagct tgatccctg
   19
<210> 17
   <211> 21
   <212> DNA
   <213> Artificial Sequence
<220>
   <223> Description of Artificial Sequence: Primer sequence GNT21F
<400> 17
   atggcagata tggctcgatt g
   21
<210> 18
   <211> 22
   <212> DNA
   <213> Artificial Sequence
<220>
   <223> Description of Artificial Sequence: Primer sequence GNT15R
<400> 18
   agtttctatg gtatctaact gc
   22
<210> 19
   <211> 21
   <212> DNA
   <213> Artificial Sequence
<220>
   <223> Description of Artificial Sequence: Primer sequence GNTHT7
<400> 19
   gagcatccaa gcttgacctg g
   21
<210> 20
   <211> 22
   <212> DNA
   <213> Artificial Sequence
<220>
   <223> Description of Artificial Sequence: Primer sequence GNTET7
<400> 20
   gcaccgtgaa ttcttctagc tt
   22
<210> 21
   <211> 23
   <212> DNA
   <213> Artificial Sequence
<220>
   <223> Description of Artificial Sequence: Primer sequence GNTHT3
<400> 21
   ggaagaacaa gcttcaaagt ggc
   23
<210> 22
   <211> 21
   <212> DNA
   <213> Artificial Sequence
<220>
   <223> Description of Artificial Sequence: Primer sequence GNTPT3
<400> 22
   gatccctgcagatctcaaacg 23
<210> 23
   <211> 19
   <212> DNA
   <213> Artificial Sequence
<220>
   <223> Description of Artificial Sequence: Primer sequence GNT7F
<400> 23
   gttcsatggt ttgagcagg
   19
<210> 24
   <211> 19
   <212> DNA
   <213> Artificial Sequence
<220>
   <223> Description of Artificial Sequence: Primer sequence GNT8R
<400> 24
   gcgacctttc ctattctcc
   19
<210> 25
   <211> 20
   <212> DNA
   <213> Artificial Sequence
<220>
   <223> Description of Artificial Sequence: Primer sequence N1
<400> 25
   taccgacagt ggtcccaaag
   20
<210> 26
   <211> 20
   <212> DNA
   <213> Artificial Sequence
<220>
   <223> Description of Artificial Sequence: Primer sequence N2
<400> 26
   ccaccatgat attcggcaag
   20
<210> 27
   <211> 20
   <212> DNA
   <213> Artificial Sequence
<220>
   <223> Description of Artificial Sequence: Primer sequence GNT5F
<400> 27
   tgggctttaa cacaactttt
   20
<210> 28
   <211> 19
   <212> DNA
   <213> Artificial Sequence
<220>
   <223> Description of Artificial Sequence: Primer sequence N3
<400> 28
   tgtcgtgctc caccatgtt
   19
<210> 29
   <211> 18
   <212> DNA
   <213> Artificial Sequence
<220>
   <223> Description of Artificial Sequence: Primer sequence N4
<400> 29
   gttgagcata taagaaac
   18
<210> 30
   <211> 22
   <212> DNA
   <213> Artificial Sequence
<220>
   <223> Description of Artificial Sequence: Primer sequence GNT10R
<400> 30
   cacattgttc aatttgatag ac
   22
<210> 31
   <211> 21
   <212> DNA
   <213> Artificial Sequence
<220>
   <223> Description of Artificial Sequence: Primer sequence FD4F
<400> 31
   tgggcngart aygayatgat g
   21
<210> 32
   <211> 23
   <212> DNA
   <213> Artificial Sequence
<220>
   <223> Description of Artificial Sequence: Primer sequence FDR1
<400> 32
   tgngtnarnc cnadnggrta dat
   23
<210> 33
   <211> 17
   <212> DNA
   <213> Artificial Sequence
<220>
   <223> Description of Artificial Sequence: Primer sequence FD5R
<400> 33
   tgnacngcng ccatrtc
   17
<210> 34
   <211> 18
   <212> DNA
   <213> Artificial Sequence
<220>
   <223> Description of Artificial Sequence: Primer sequence 5FT4
<400> 34
   gtaacattcg cataatgg
   18
<210> 35
   <211> 19
   <212> DNA
   <213> Artificial Sequence
<220>
   <223> Description of Artificial Sequence: Primer sequence 5FT5
<400> 35
   cgatcattat gcgcaccac
   19
<210> 36
   <211> 18
   <212> DNA
   <213> Artificial Sequence
<220>
   <223> Description of Artificial Sequence: Primer sequence 5FT6
<400> 36
   ggaaataaaa gcagctcc
   18
<210> 37
   <211> 19
   <212> DNA
   <213> Artificial Sequence
<220>
   <223> Description of Artificial Sequence: Primer sequence 5FT7
<400> 37
   agggtgaatc tccatagcc
   19
<210> 38
   <211> 19
   <212> DNA
   <213> Artificial Sequence
<220>
   <223> Description of Artificial Sequence: Primer sequence 5FT8
<400> 38
   catctgcctg accctcacc
   19
<210> 39
   <211> 18
   <212> DNA
   <213> Artificial Sequence
<220>
   <223> Description of Artificial Sequence: Primer sequence 5FT9
<400> 39
   gccttgaaca cgcatggc
   18
<210> 40
   <211> 18
   <212> DNA
   <213> Artificial Sequence
<220>
   <223> Description of Artificial Sequence: Primer sequence 5FT10
<400> 40
   cgatacaacc agcacagg
   18
<210> 41
   <211> 19
   <212> DNA
   <213> Artificial Sequence
<220>
   <223> Description of Artificial Sequence: Primer sequence 5FT11
<400> 41
   cttctctagc cattctgcc
   19
<210> 42
   <211> 20
   <212> DNA
   <213> Artificial Sequence
<220>
   <223> Description of Artificial Sequence: Primer sequence 3FT1
<400> 42
   gcagtggaag tttaatggtc
   20
<210> 43
   <211> 21
   <212> DNA
   <213> Artificial Sequence
<220>
   <223> Description of Artificial Sequence: Primer sequence 3FT2
<400> 43
   tcgtttctag ctctagtaga c
   21
<210> 44
   <211> 18
   <212> DNA
   <213> Artificial Sequence
<220>
   <223> Description of Artificial Sequence: Primer sequence FTA9F
<400> 44
   atgctcccag cccaagac
   18
<210> 45
   <211> 21
   <212> DNA
   <213> Artificial Sequence
<220>
   <223> Description of Artificial Sequence: Primer sequence FTA10R
<400> 45
   tgtctactag agctagaaac g
   21
<210> 46
   <211> 20
   <212> DNA
   <213> Artificial Sequence
<220>
   <223> Description of Artificial Sequence: Primer sequence FT18F
<400> 46
   tagggagtaa atatgaaggg
   20
<210> 47
   <211> 21
   <212> DNA
   <213> Artificial Sequence
<220>
   <223> Description of Artificial Sequence: Primer sequence FTA12R
<400> 47
   tacttccaat tgaagacaag g
   21
<210> 48
   <211> 18
   <212> DNA
   <213> Artificial Sequence
<220>
   <223> Description of Artificial Sequence: Primer sequence FT15F
<400> 48
   aatgttctgt gccatgcg
   18
<210> 49
   <211> 19
   <212> DNA
   <213> Artificial Sequence
<220>
   <223> Description of Artificial Sequence: Primer sequence FT16R
<400> 49
   tgcttcaaat gggctaggg
   19
<210> 50
   <211> 28
   <212> DNA
   <213> Artificial Sequence
<220>
   <223> Description of Artificial Sequence: Primer sequence nptII/NdeI-F
<400> 50
   atgccatatg gcatgcctgc aggtcaac
   28
<210> 51
   <211> 29
   <212> DNA
   <213> Artificial Sequence
<220>
   <223> Description of Artificial Sequence: Primer sequence nptII/BstZ17I-R
<400> 51
   gcatgtatac gcatgcctgc aggtcactg
   29
<210> 52
   <211> 19
   <212> DNA
   <213> Artificial Sequence
<220>
   <223> Description of Artificial Sequence: Primer sequence FT14F
<400> 52
   acaaagttac atactcgcg
   19
<210> 53
   <211> 20
   <212> DNA
   <213> Artificial Sequence
<220>
   <223> Description of Artificial Sequence: Primer sequence R10
<400> 53
   tctttcacta ttcggtgacg
   20
<210> 54
   <211> 19
   <212> DNA
   <213> Artificial Sequence
<220>
   <223> Description of Artificial Sequence: Primer sequence R11
<400> 54
   cgaccacaac attagatcc
   19
<210> 55
   <211> 23
   <212> DNA
   <213> Artificial Sequence
<220>
   <223> Description of Artificial Sequence: Primer sequence XDF1
<400> 55
   tgygargsnt ayttyggnaa ygg
   23
<210> 56
   <211> 23
   <212> DNA
   <213> Artificial Sequence
<220>
   <223> Description of Artificial Sequence: Primer sequence XDR1
<400> 56
   gcncknayca tytcnccraa ytc
   23
<210> 57
   <211> 23
   <212> DNA
   <213> Artificial Sequence
<220>
   <223> Description of Artificial Sequence: Primer sequence XDF2
<400> 57
   ggnggngara arytngarra ngt
   23
<210> 58
   <211> 18
   <212> DNA
   <213> Artificial Sequence
<220>
   <223> Description of Artificial Sequence: Primer sequence 5XT1
<400> 58
   tcctccttct ctgggacc
   18
<210> 59
   <211> 21
   <212> DNA
   <213> Artificial Sequence
<220>
   <223> Description of Artificial Sequence: Primer sequence 5XT2
<400> 59
   agctccagtt gtgaaatatg g
   21
<210> 60
   <211> 18
   <212> DNA
   <213> Artificial Sequence
<220>
   <223> Description of Artificial Sequence: Primer sequence 5XT4
<400> 60
   cttccttcac cacactac
   18
<210> 61
   <211> 18
   <212> DNA
   <213> Artificial Sequence
<220>
   <223> Description of Artificial Sequence: Primer sequence 5XT5
<400> 61
   tagcatgact gtgtggcc
   18
<210> 62
   <211> 18
   <212> DNA
   <213> Artificial Sequence
<220>
   <223> Description of Artificial Sequence: Primer sequence 5XT6
<400> 62
   aaaggcttga gtgtagcc
   18
<210> 63
   <211> 19
   <212> DNA
   <213> Artificial Sequence
<220>
   <223> Description of Artificial Sequence: Primer sequence 3XT1
<400> 63
   gcctttcttg cacgggttg
   19
<210> 64
   <211> 19
   <212> DNA
   <213> Artificial Sequence
<220>
   <223> Description of Artificial Sequence: Primer sequence 3XT2
<400> 64
   ggacattcca aataatccc
   19
<210> 65
   <211> 19
   <212> DNA
   <213> Artificial Sequence
<220>
   <223> Description of Artificial Sequence: Primer sequence XT15R
<400> 65
   gtcctgttaa atgccttgc
   19
<210> 66
   <211> 20
   <212> DNA
   <213> Artificial Sequence
<220>
   <223> Description of Artificial Sequence: Primer sequence XT-M1F
<400> 66
   aggttgagca atcatatggc
   20
<210> 67
   <211> 20
   <212> DNA
   <213> Artificial Sequence
<220>
   <223> Description of Artificial Sequence: Primer sequence XT11R
<400> 67
   atcccagaaa tatctgatcc
   20
<210> 68
   <211> 18
   <212> DNA
   <213> Artificial Sequence
<220>
   <223> Description of Artificial Sequence: Primer sequence XT12F
<400> 68
   tgtgaggcgt tctttggc
   18
<210> 69
   <211> 29
   <212> DNA
   <213> Artificial Sequence
<220>
   <223> Description of Artificial Sequence: Primer sequence nptII/SalI-F
<400> 69
   atgcgtcgac gtcaacatgg tggagcacg
   29
<210> 70
   <211> 31
   <212> DNA
   <213> Artificial Sequence
<220>
   <223> Description of Artificial Sequence: Primer sequence nptII/NdeI-R
<400> 70
   gcatcatatg tcactggatt ttggttttag g
   31
<210> 71
   <211> 20
   <212> DNA
   <213> Artificial Sequence
<220>
   <223> Description of Artificial Sequence: Primer sequence XT13R
<400> 71
   acgatccaaa atctggacgc
   20
<210> 72
   <211> 32
   <212> DNA
   <213> Artificial Sequence
<220>
   <223> Description of Artificial Sequence: Primer sequence GalTXh-F
<400> 72
   ttctcgagac aatgaggctt cgggagccgc tc
   32
<210> 73
   <211> 30
   <212> DNA
   <213> Artificial Sequence
<220>
   <223> Description of Artificial Sequence: Primer sequence GalTXb-R
<400> 73
   ggtctagact agctcggtgt cccgatgtcc
   30
<210> 74
   <211> 30
   <212> DNA
   <213> Artificial Sequence
<220>
   <223> Description of Artificial Sequence: Primer sequence XTB-F
<400> 74
   ttggatcctc aattacgaag cacaccatgc
   30
<210> 75
   <211> 32
   <212> DNA
   <213> Artificial Sequence
<220>
   <223> Description of Artificial Sequence: Primer sequence XTB-R
<400> 75
   ttggatcctc ctcccagaaa catctgatcc ag
   32
<210> 76
   <211> 28
   <212> DNA
   <213> Artificial Sequence
<220>
   <223> Description of Artificial Sequence: Primer sequence MoB521
<400> 76
   ttgccgctat ctacttgtat gctaacgt
   28
<210> 77
   <211> 25
   <212> DNA
   <213> Artificial Sequence
<220>
   <223> Description of Artificial Sequence: Primer sequence MoB575
<400> 77
   tgccgtggat gtgctagata atctt
   25
<210> 78
   <211> 33
   <212> DNA
   <213> Artificial Sequence
<220>
   <223> Description of Artificial Sequence: Primer sequence FTB-F
<400> 78
   taggatccag atgatgtctg ctcggcagaa tgg
   33
<210> 79
   <211> 33
   <212> DNA
   <213> Artificial Sequence
<220>
   <223> Description of Artificial Sequence: Primer sequence FTB-R
<400> 79
   ctggatcctt gtagatccga aggtctgagt tcc
   33
<210> 80
   <211> 26
   <212> DNA
   <213> Artificial Sequence
<220>
   <223> Description of Artificial Sequence: Primer sequence MoB435
<400> 80
   tcctacctgc ggagcaacag atattg
   26
<210> 81
   <211> 27
   <212> DNA
   <213> Artificial Sequence
<220>
   <223> Description of Artificial Sequence: Primer sequence MoB495
<400> 81
   gtggacccag atttgctggt gcacttg
   27
<210> 82
   <211> 18
   <212> DNA
   <213> Artificial Sequence
<220>
   <223> Description of Artificial Sequence: Primer sequence XT14F
<400> 82
   ttacgaagca caccatgc
   18
<210> 83
   <211> 19
   <212> DNA
   <213> Artificial Sequence
<220>
   <223> Description of Artificial Sequence: Primer sequence 5XT3
<400> 83
   ttcttcctca tttcgtccc
   19

## Claims

1. A transformed bryophyte cell that comprises a double knockout of fucosyl transferase and xylosyl transferase that results in modified N-linked glycans without detectable 1,3-linked fucosyl and 1,2-linked xylosyl residues.

2. A transformed bryophyte cell according to claim 1, wherein the said cell further comprises a nucleotide sequence operably linked to an exogenous promoter that drives expression in the said bryophyte cell, wherein said nucleotide sequence encodes a glycosylated polypeptide that is expressed in the bryophyte cell.

3. A transformed bryophyte cell according to claim 2, wherein said glycosylated polypeptide comprises animal glycosylation patterns.

4. A transformed bryophyte cell according to claim 3, wherein said glycosylated polypeptide comprises mammalian glycosylation patterns.

5. A transformed bryophyte cell according to any one of claims 1 to 4, further comprising a nucleotide sequence operably linked to an exogenous promoter that drives expression in the said bryophyte cell, wherein said nucleotide sequence encodes a functional mammalian galactosyl transferase that is expressed in the bryophyte cell.

6. A transformed bryophyte cell according to claim 5, wherein the mammalian galactosyl transferase that is expressed is a beta-1,4 galT.

7. A transformed bryophyte cell according to claim 6, wherein the mammalian galactosyl transferase that is expressed is a human beta-1,4 galT.

8. A bryophyte cell according to any one of claims 1 to 7, wherein the bryophyte cell is selected from species of the genera *Physcomitrella, Funaria, Sphagnum, Ceratodon, Marchantia* and *Sphaerocarpos.*

9. A bryophyte cell according to claim 8, wherein the bryophyte cell is selected from *Physcomitrella.*

10. A bryophyte cell according to claim 9, wherein the bryophyte cell is from *Physcomitrella patens.*

11. A bryophyte cell according to any one of claims 2 to 10, wherein the glycosylated polypeptide is selected from the group comprising a polypeptide having a primary amino acid sequence of a human glycosylated polypeptide, a primary amino acid sequence of a non-human mammalian glycosylated protein, a primary amino acid sequence of an antibody or an active fragment thereof, and/or a primary amino acid sequence of a non-mammalian glycosylated polypeptide.

12. A bryophyte cell according to claim 11, wherein the glycosylated polypeptide is a human polypeptide.

13. A bryophyte cell according co claim 11 or 12, wherein the glycosylated polypeptide is selected from the group consisting of human insulin, preproinsulin, VEGF, proinsulin, glucagon, interferons such as alpha-interferon, beca-interferon, ganama-interferon, blood-clotting factors selected from Factor VII, VIII, IX, X, XI, and XII, fertility hormones including luteirising hormone, follicle stimulating hormone, growth factors including epidermal growth factor, platelet-derived growth factor, granulocyte colony stimulating factor prolactin, oxytocin, thyroid stimulating hormone, adrenocorticotropic hormone, calcitonin, parathyroid hormone, somatostatin, erythropoietin (EPO), and enzymes such as betaglucocerebrosidase, haemoglobin, serum albumin, and collagen.

14. A method of producing at least a bryophyte cell that comprises a double knockout of fucosyl transferase and xylosyl transferase that results in modified N-linked glycans without detectable 1,3-linked fucosyl and 1,2-linked xylosyl residues, that comprises introducing into the said cell i) a first nucleic acid sequence that is specifically targeted to an endogenous fucosyl transferase nucleotide sequence and ii) introducing into the said cell a second nucleic acid sequence that is specifically targeted to an endogenous xylosyl transferase nucleotide sequence.

15. A method according to claim 14, wherein the said transformed bryophyte cell further comprises a nucleotide sequence operably linked to an exogenous promoter that drives expression in the said bryophyte cell, wherein said nucleotide sequence encodes a glycosylated polypeptide that is expressed in the bryophyte cell.

16. A method according to claim 15, wherein said glycosylated polypeptide comprises animal glycosylation patterns.

17. A method according to claim 16, wherein said glycosylated polypeptide comprises mammalian glycosylation patterns.

18. A method according to any one of claims 14 to 17, further comprising introducing into the said cell an isolated nucleic acid sequence that comprises nucleic acid operably linked to an exogenous promoter that drives expression in a bryophyte cell, wherein said nucleic acid encodes a functional mammalian galactosyl transferase polypeptide.

19. A method according to claim 18, wherein the galactosyl transferase nucleotide sequence is a beta-1,4 galactosyl transferase (beta-1,4 galT) nucleotide sequence.

20. A method according to claim 19, wherein the galactosyl transferase nucleotide sequence is a human beta-1,4 galactosyl transferase (beta-1,4 galT) nucleotide sequence.

21. A method according to any one of claims 15 to 20, wherein the glycosylated polypeptide is selected from the group comprising a protein having a primary amino acid sequence of a human protein, a primary amino acid sequence of a non-human mammalian protein, a primary amino acid sequence of an antibody or an active fragment thereof, and/or a primary amino acid sequence of a non-mammalian protein.

22. A method according to any one of claims 15 co 21, wherein che glycosylated polypeptide is selected from the group consisting of human insulin, prepoinsulin, VEGF, proinsulin, glucagon, interferons such as alpha- interferon, beta-interferon, gamma-interferon, blood-clotting factors selected from Factor VII, VIII, IX, X, XI, and XII, fertility hormones including luteinising hormone, follicle stimulating hormone, growth factors including epidermal growth factor, platelet-derived growth factor, granulocyte colony stimulating factor, prolactin, oxytocin, thyroid stimulating hormone, adrenocorticotropic hormone, calcitonin, parathyroid hormone, somatostatin, erythropoietin (EPO), and enzymes such as betaglucocerebrosidase, haemoglobin, serum albumin, collagen, and human and non-human proteins selected from amidases, amylases, carbohydrases, cellulase, dextranase, esterases, glucanases, glucoamylase, lactase, lipases, pepsin, peptidases, phytases, proteases, pectinases, casein, whey proteins, soya proteins, gluten and egg albumin.

23. A method according to any one of claims 14 to 22, wherein the bryophyte cell is selected from species of the genera *Physcomitrella, Funaria, Sphagnum, Ceratodon, Marchantia* and *Sphaerocarpos.*

24. A method according to claim 23, wherein the bryophyte cell is selected from *Physcomitrella.*

25. A method according to claim 24, wherein the bryophyte cell is from *Physcomitrella patens.*

26. A method according to any one of claims 15 to 25, wherein the glycosylated polypeptide is selected from the group comprising a polypeptide having a primary amino acid sequence of a human glycosylated polypeptide, a primary amino acid sequence of a non-human mammalian glycosylated protein, a primary amino acid sequence of an antibody or an active fragment thereof, and/or a primary amino acid sequence of a non-mammalian glycosylated polypeptide.

27. A method according to claim 26, wherein the glycosylated polypeptide is a human polypeptide.

28. A method according to claim 26 or 27, wherein the glycosylated polypeptide is selected from the group consisting of human insulin, preproinsulin, VEGF, proinsulin, glucagon, interferons such as alpha-interferon, beta-interferon, gamma-interferon, blood-clotting factors selected from Factor VII, VIII, IX, X, XI, and XII, fertility hormones including luteinising hormone, follicle stimulating hormone growth factors including epidermal growth factor, platelet-derived growth factor, granulocyte colony stimulating factor, prolactin, oxytocin, thyroid stimulating hormone, adrenocorticotropic hormone, calcitonin, parathyroid hormone, somatostatin, erythropoietin (EPO), and enzymes such as betaglucocerebrosidase, haemoglobin, serum albumin, and collagen.

29. A method according to any one of claims 15 to 28, wherein the exogenous promoter is selected from inducible, chemical-regulated, constitutive or cell specific promoters.

30. A set of nucleic acid vectors suitable for producing at least a transformed bryophyte cell that comprises a double knockout of fucosyl transferase and xylosyl transferase that results in modified N-linked glycans without detectable 1,3-linked fucosyl and 1,2-linked xylosyl residues, wherein said set of nucleic acid vectors comprises i) a first nucleic acid sequence that is specifically targeted to an endogenous fucosyl transferase nucleotide sequence and ii) a second nucleic acid sequence that is specifically targeted to an endogenous xylosyl transferase nucleotide sequence.

31. A set of nucleic acid vectors according to claim 30, further including a polynucleotide that encodes a glycosylated polypeptide.

32. A set of nucleic acid vectors according to claim 30 or 31, further including a polynucleotide that encodes a functional mammalian glycosyl transferase for use in a method according to any of claims 15 to 29.

33. A set of nucleic acid vectors according to claim 32, wherein said polynucleotide encodes a mammalian galactosyl transferase.

34. A set of nucleic acid vectors according to claim 33, wherein said polynucleotide encodes a human beta-1,4 galactosyl transferase.

35. A host cell containing the set of nucleic acid vectors according to any one of claims 30 to 34.

36. A host cell according to claim 35 which is a bryophyte cell.

37. A host cell according to claim 35 which is a prokaryote cell.

38. A method of producing a host cell according to any of claims 35 to 37, the method including incorporating said set of nucleic acid vectors into the cell by means of transformation.

39. Use of the set of nucleic acid vectors according to any one of claims 30 to 34 in the production of a transgenic bryophyte cell.

40. A host cell according to claim 35 or 36 which is comprised in a bryophyte, or a bryophyte part, or an extract or derivative of a bryophyte or in a bryophyte cell culture.

41. A bryophyte plant or bryophyte tissue comprising a bryophyte cell according to any one of claims 1 to 13, 36 and 40.

42. A method of producing a bryophyte plant, the method including incorporating the set of nucleic acid vectors according to any of claims 30 to 34 into a bryophyte cell and regenerating a bryophyte from said cell.

## Patentansprüche

1. Transformierte Mooszelle, die ein Doppel-Knockout von Fucosyl-Transferase und Xylosyl-Transferase umfasst, woraus modifizierte N-gebundene Glykane ohne nachweisbare 1,3-gebundene Fucosyl- und 1,2-gebundene Xylosylreste resultieren.

2. Transformierte Mooszelle nach Anspruch 1, wobei die Zelle ferner eine Nukleotidsequenz umfasst, die funktionsfähig mit einem exogenen Promotor verknüpft ist, der die Expression in der Mooszelle steuert, wobei die Nukleotidsequenz ein glykosyliertes Polypeptid kodiert, das in der Mooszelle exprimiert wird.

3. Transformierte Mooszelle nach Anspruch 2, wobei das glykosylierte Polypeptid tierische Glykosylierungsmuster umfasst.

4. Transformierte Mooszelle nach Anspruch 3, wobei das glykosylierte Polypeptid Säuger-Glykosylierungsmuster umfasst.

5. Transformierte Mooszelle nach irgendeinem der Ansprüche 1 bis 4, ferner umfassend eine Nukleotidsequenz, die funktionsfähig mit einen exogenen Promotor verknüpft ist, der die Expression in der Mooszelle steuert, wobei die Nukleotidsequenz eine funktionale Säuger-Galaktosyltransferase kodiert, die in der Mooszelle exprimiert wird.

6. Transformierte Mooszelle nach Anspruch 5, wobei die exprimierte Säuger-Galaktosyltransferase eine Beta-1,4 galT ist.

7. Transformierte Mooszelle nach Anspruch 6, wobei die exprimierte Säuger-Galaktosyltransferase eine humane Beta-1,4 galT ist.

8. Mooszelle nach irgendeinem der Ansprüche 1 bis 7, wobei die Mooszelle ausgewählt ist aus Spezies der Gattungen Physcomitrella, Funaria, Sphagnum, Ceratodon, Marchantia und Sphaerocarpos.

9. Mooszelle nach Anspruch 8, wobei die Mooszelle aus Physcomitrella ausgewählt ist.

10. Mooszelle nach Anspruch 9, wobei die Mooszelle von Physcomitrella patens ist.

11. Mooszelle nach irgendeinem der Ansprüche 2 bis 10, wobei das glykosylierte Polypeptid ausgewählt ist aus der Gruppe umfassend ein Polypeptid mit einer primären Aminosäuresequenz eines humanen glykosylierten Polypeptids, einer primären Aminosäuresequenz eines nicht-humanen glykosylierten Säugerproteins, einer primären Aminosäuresequenz eines Antikörpers oder eines aktiven Fragments davon, und/oder einer primären Aminosäuresequenz eines glykosylierten Nicht-Säugerpolypeptids.

12. Mooszelle nach Anspruch 11, wobei das glykosylierte Polypeptid ein humanes Polypeptid ist.

13. Mooszelle nach Anspruch 11 oder 12, wobei das glykosylierte Polypeptid ausgewählt ist aus der Gruppe bestehend aus humanem Insulin, Präproinsulin, VEGF, Proinsulin, Glukagon, Interferonen wie Alpha-Interferon, Beta-Interferon, Gamma-Interferon, Blutgerinnungsfaktoren ausgewählt aus Faktor VII, VIII, IX, X, XI, und XII, Fertilitätshormonen einschließlich luteinisierendem Hormon, follikelstimulierendem Hormon, Wachstumsfaktoren einschließlich epidermaler Wachstumsfaktor, PDGF, Granulozyten-Kolonie-stimulierender Faktor, Prolaktin, Oxytocin, Thyroid-stimulierendem Hormon, adrenokortikotropem Hormon, Calcitonin, Parathyroid-Hormon, Somatostatin, Erythropoietin (EPO), und Enzymen wie Beta-Glukocerebrosidase, Hämoglobin, Serumalbumin und Kollagen.

14. Verfahren zur Herstellung mindestens einer Mooszelle, die ein Doppel-Knockout von Fucosyl-Transferase und Xylosyl-Transferase umfasst, woraus modifizierte N-gebundene Glykane ohne nachweisbare 1,3-gebundene Fucosyl- und 1,2-gebundene Xylosylreste resultieren, umfassend das Einführen in die Zelle von i) einer ersten Nukleinsäuresequenz, die spezifisch auf eine endogene Fucosyltransferase-Nukleotidsequenz gerichtet ist und ii) das Einführen in die Zelle einer zweiten Nukleinsäuresequenz, die spezifisch auf eine endogene Xylosyltransferase-Nukleotidsequenz gerichtet ist.

15. Verfahren nach Anspruch 14, wobei die transformierte Mooszelle ferner eine Nukleotidsequenz umfasst, die funktionsfähig mit einem exogenen Promotor verknüpft ist, der die Expression in der Mooszelle steuert, wobei die Nukleotidsequenz ein glykosyliertes Polypeptid kodiert, das in der Mooszelle exprimiert wird.

16. Verfahren nach Anspruch 15, wobei das glykosylierte Polypeptid tierische Glykosylierungsmuster umfasst.

17. Verfahren nach Anspruch 16, wobei das glykosylierte Polypeptid Säuger-Glykosylierungsmuster umfasst.

18. Verfahren nach irgendeinem der Ansprüche 14 bis 17, ferner umfassend das Einführen in die Zelle einer isolierten Nukleinsäuresequenz, welche eine Nukleinsäure umfasst, die funktionsfähig mit einem exogenen Promotor verknüpft ist, der die Expression in einer Mooszelle steuert, wobei die Nukleinsäure ein funktionales Säuger-Galaktosyltransferase-Polypeptid kodiert.

19. Verfahren nach Anspruch 18, wobei die Galaktosyltransferase-Nukleotidsequenz eine Beta-1,4 Galaktosyltransferase (Beta-1,4 galT)-Nukleotidsequenz ist.

20. Verfahren nach Anspruch 19, wobei die Galaktosyltransferase-Nukleotidsequenz eine humane Beta-1,4 Galaktosyltransferase (Beta-1,4 galT)-Nukleotidsequenz ist.

21. Verfahren nach irgendeinem der Ansprüche 15 bis 20, wobei das glykosylierte Polypeptid ausgewählt wird aus der Gruppe umfassend ein Protein mit einer primären Aminosäuresequenz eines humanen Proteins, einer primären Aminosäuresequenz eines nicht-humanen Säugerproteins, einer primären Aminosäuresequenz eines Antikörpers oder eines aktiven Fragments davon, und/oder einer primären Aminosäuresequenz eines Nicht-Säugerproteins.

22. Verfahren nach irgendeinem der Ansprüche 15 bis 21, wobei das glykosylierte Polypeptid ausgewählt wird aus der Gruppe bestehend aus humanem Insulin, Präproinsulin, VEGF, Proinsulin, Glukagon, Interferonen wie Alpha-Interferon, Beta-Interferon, Gamma-Interferon, Blutgerinnungsfaktoren ausgewählt aus Faktor VII, VIII, IX, X, XI, und XII, Fertilitätshormonen einschließlich luteinisierendem Hormon, follikelstimulierendem Hormon, Wachstumsfaktoren einschließlich epidermaler Wachstumsfaktor, PDGF, Granulozyten-Kolonie-stimulierender Faktor, Prolaktin, Oxytocin, Thyroid-stimulierendem Hormon, adrenokortikotropem Hormon, Calcitonin, Parathyroid-Hormon, Somatostatin, Erythropoietin (EPO), und Enzymen wie Beta-Glukocerebrosidase, Hämoglobin, Serumalbumin, Kollagen, und humanen sowie nicht-humanen Proteinen ausgewählt aus Amidasen, Amylasen, Carbohydrasen, Cellulase, Dextranase, Esterasen, Glucanasen, Glucoamylase, Lactase, Lipasen, Pepsin, Peptidasen, Phytasen, Proteasen, Pektinasen, Kasein, Molkeproteinen, Sojaproteinen, Gluten und Ovalbumin.

23. Verfahren nach irgendeinem der Ansprüche 14 bis 22, wobei die Mooszelle ausgewählt wird aus Spezies der Gattungen Physcomitrella, Funaria, Sphagnum, Ceratodon, Marchantia und Sphaerocarpos.

24. Verfahren nach Anspruch 23, wobei die Mooszelle aus Physcomitrella ausgewählt wird.

25. Verfahren nach Anspruch 24, wobei die Mooszelle von Physcomitrella patens ist.

26. Verfahren nach irgendeinem der Ansprüche 15 bis 25, wobei das glykosylierte Polypeptid ausgewählt wird aus der Gruppe umfassend ein Polypeptid mit einer primären Aminosäuresequenz eines humanen glykosylierten Polypeptids, einer primären Aminosäuresequenz eines nicht-humanen glykosylierten Säugerproteins, einer primären Aminosäuresequenz eines Antikörpers oder eines aktiven Fragments davon, und/oder einer primären Aminosäuresequenz eines glykosylierten Nicht-Säugerpolypeptids.

27. Verfahren nach Anspruch 26, wobei das glykosylierte Polypeptid ein humanes Polypeptid ist.

28. Verfahren nach Anspruch 26 oder 27, wobei das glykosylierte Polypeptid ausgewählt wird aus der Gruppe bestehend aus humanem Insulin, Präproinsulin, VEGF, Proinsulin, Glukagon, Interferonen wie Alpha-Interferon, Beta-Interferon, Gamma-Interferon, Blutgerinnungsfaktoren ausgewählt aus Faktor VII, VIII, IX, X, XI, und XII, Fertilitätshormonen einschließlich luteinisierendem Hormon, follikelstimulierendem Hormon, Wachstumsfaktoren einschließlich epidermaler Wachstumsfaktor, PDGF, Granulozyten-Kolonie-stimulierender Faktor, Prolaktin, Oxytocin, Thyroid-stimulierendem Hormon, adrenokortikotropem Hormon, Calcitonin, Parathyroid-Hormon, Somatostatin, Erythropoietin (EPO), und Enzymen wie BetaGlucocerebrosidase, Hämoglobin, Serumalbumin und Kollagen.

29. Verfahren nach irgendeinem der Ansprüche 15 bis 28, wobei der exogene Promotor aus induzierbaren, chemisch regulierten, konstitutiven oder zellspezifischen Promotoren ausgewählt wird.

30. Satz von Nukleinsäurevektoren, die geeignet sind zur Herstellung mindestens einer transformierten Mooszelle, die ein Doppel-Knockout von Fucosyl-Transferase und Xylosyl-Transferase umfasst, woraus modifizierte N-gebundene Glykane ohne nachweisbare 1,3-gebundene Fucosyl- und 1,2-gebundene Xylosylreste resultieren, wobei der Satz von Nukleinsäurevektoren i) eine erste Nukleinsäuresequenz, die spezifisch auf eine endogene Fucosyltransferase-Nukleotidsequenz gerichtet ist, und ii) eine zweite Nukleinsäuresequenz umfasst, die spezifisch auf eine endogene Xylosyltransferase-Nukleinsäuresequenz gerichtet ist.

31. Satz von Nukleinsäurevektoren nach Anspruch 30, ferner umfassend ein Polynukleotid, das ein glykosyliertes Polypeptid kodiert.

32. Satz von Nukleinsäurevektoren nach Anspruch 30 oder 31, ferner einschließend ein Polynukleotid, das eine funktionale Säuger-Glykosyltransferase kodiert, zur Verwendung in einem Verfahren gemäß irgendeinem der Ansprüche 15 bis 29.

33. Satz von Nukleinsäurevektoren nach Anspruch 32, wobei das Polynukleotid eine Säuger-Galaktosyltransferase kodiert.

34. Satz von Nukleinsäurevektoren nach Anspruch 33, wobei das Polynukleotid eine humane Beta-1,4 Galaktosyltransferase kodiert.

35. Wirtszelle, die den Satz von Nukleinsäurevektoren gemäß irgendeinem der Ansprüche 30 bis 34 enthält.

36. Wirtszelle nach Anspruch 35, die eine Mooszelle ist.

37. Wirtszelle nach Anspruch 35, die eine Prokaryontenzelle ist.

38. Verfahren zur Herstellung einer Wirtszelle gemäß irgendeinem der Ansprüche 35 bis 37, wobei das Verfahren das Einführen des Satzes von Nukeinsäurevektoren in die Zelle mittels Transformation einschließt.

39. Verwendung des Satzes von Nukleinsäurevektoren gemäß irgendeinem der Ansprüche 30 bis 34 bei der Herstellung einer transgenen Mooszelle.

40. Wirtszelle nach Anspruch 35 oder 36, welche von einem Moos oder einem Moosteil oder einem Extrakt oder Derivat eines Mooses oder einer Mooszellkultur umfasst ist.

41. Moospflanze oder Moosgewebe, umfassend eine Mooszelle gemäß irgendeinem der Ansprüche 1 bis 13, 36 und 40.

42. Verfahren zur Herstellung einer Moospflanze, wobei das Verfahren das Einführen des Satzes von Nukleinsäurevektoren nach irgendeinem der Ansprüche 30 bis 34 in eine Mooszelle und das Regenerieren eines Mooses aus dieser Zelle einschließt.

## Revendications

1. Une cellule de bryophyte transformée qui contient un double Knock-out de la transférase de fucosyle et de la transférase de xylosyle, **caractérisée en ce qu'** elle contient des glycanes liés par N (N-Linked) modifiés sans résidus fucosyle 1,3-Linked et xylosyle 1,2-Linked détectables.

2. Une cellule de bryophyte transformée selon la revendication 1, **caractérisée en ce que** la dite cellule contient de plus une séquence nucléotidique, qui est liée de manière opérationnelle à un promoteur exogène s'exprimant dans la dite cellule de bryophyte, la dite séquence nucléotidique codant pour un polypeptide glycosylé qui est exprimée dans la cellule de bryophyte.

3. Une cellule de bryophyte transformée selon la revendication 2, **caractérisée en ce que** le dit polypeptide glycosylé contient des motifs de glycosylation d'animal.

4. Une cellule de bryophyte transformée selon la revendication 3, **caractérisée en ce que** le dit polypeptide glycosylé contient des motifs de glycosylation de mammifère.

5. Une cellule de bryophyte transformée selon une des revendications 1 à 4, qui contient de plus une séquence nucléotidique liée de manière opérationnelle à un promoteur exogène s'exprimant dans la dite cellule de bryophyte, la dite séquence nucléotidique codant pour une transférase de galactosyle de mammifère fonctionnelle qui est exprimée dans la cellule de bryophyte.

6. Une cellule de bryophyte transformée selon la revendication 5, **caractérisée en ce que** la transférase de galactosyle de mammifère qui est exprimée, est une beta-1,4 galT.

7. Une cellule de bryophyte transformée selon la revendication 6, **caractérisée en ce que** la transférase de galactosyle de mammifère qui est exprimée, est une beta-1,4 galT humaine.

8. Une cellule de bryophyte selon une des revendications 1 à 7, **caractérisée en ce que** la cellule de bryophyte est choisie parmi les espèces des genres *Physcomitrella, Funaria, Sphagnum, Ceratodon, Marchantia* et *Sphaerocarpos.*

9. Une cellule de bryophyte selon la revendication 8, **caractérisée en ce que** la cellule de bryophyte est choisie dans le genre *Physcomitrella.*

10. Une cellule de bryophyte selon la revendication 9, **caractérisée en ce que** la cellule de bryophyte est choisie parmi *Physcomitrella patens.*

11. Une cellule de bryophyte selon l'une des revendications 2 à 10, **caractérisée en ce que** le polypeptide glycosylé est choisi dans un groupe comprenant un polypeptide possédant une séquence primaire en acides animés correspondant à une séquence de polypeptide glycosylé humain, une séquence primaire en acides animés de polypeptide glycosylé de mammifère non humain, une séquence primaire en acides animés d'un anticorps ou d'un fragment actif de celui-ci, et/ou une séquence primaire en acides animés d'un polypeptide glycosylé de non mammifère.

12. Une cellule de bryophyte selon la revendication 11, **caractérisée en ce que** le polypeptide glycosylé est un polypeptide humain.

13. Une cellule de bryophyte selon la revendication 11 ou 12, **caractérisée en ce que** le polypeptide glycosylé est choisi dans le groupe constitué de l'insuline humaine, la préproinsuline, le VEGF, la proinsuline, le glucagon, les interférons tel que l'alpha-interféron, le béta-interféron, le gamma-interféron, les facteurs de la coagulation du sang choisis parmi les facteurs VII, VIII, IX, X, et XII, les hormones de la fertilité incluant l'hormone lutéinisante, l'hormone de stimulation des follicules, les facteurs de croissance incluant le facteur de croissance de l'épiderme, le facteur de croissance dérivé des plaquettes, le facteur de stimulation des colonies de granulocytes, la prolactine, l'oxytocine, l'hormone de stimulation de la thyroïde, l'hormone adrénocorticotropique, la calcitonine, l'hormone parathyroïde, la somatostatine, l'érythropoïétine (EPO), et des enzymes telle que la beta-glucocérébrosidase, l'hémoglobine, l'albumine sérique, et le collagène.

14. Une méthode de production d'au moins une cellule de bryophyte, comprenant un double knock-out de la transférase de fucosyle et la transférase de xylosyle, **caractérisée en ce qu'** elle contient des glycanes liés par N (N-Linked) modifiés sans résidus fucosyle 1,3-Linked et xylosyle 1,2-Linked détectables, qui comprend l'introduction dans la dite cellule de i) une première séquence d'acide nucléique qui est spécifiquement ciblée vers une séquence nucléotidique endogène de la transférase de fucosyle, et ii) l'introduction dans la dite cellule d'une seconde séquence d'acide nucléique qui est spécifiquement ciblée vers une séquence nucléotidique endogène de la transférase de xylosyle.

15. Une méthode selon la revendication 14, **caractérisée en ce que** la dite cellule de bryophyte comprend de plus une séquence nucléotidique liée de manière opérationnelle à un promoteur exogène s'exprimant dans la dite cellule de bryophyte, ladite séquence nucléotidique codant pour un polypeptide glycosylé qui est exprimé dans la cellule de bryophyte.

16. Une méthode selon la revendication 15, **caractérisée en ce que** le dit polypeptide glycosylé contient des motifs de glycosylation d'animal.

17. Une méthode selon la revendication 16, **caractérisée en ce que** le dit polypeptide glycosylé contient des motifs de glycosylation de mammifère.

18. Une méthode selon une des revendications 14 à 17, comprenant de plus l'introduction dans la dite cellule d'une séquence d'acide nucléique isolée qui comprend un acide nucléique lié de manière opérationnelle à un promoteur exogène s'exprimant dans une cellule de bryophyte, le dit acide nucléique codant pour un polypeptide fonctionnel de la transférase de galactosyle de mammifère.

19. Une méthode selon la revendication 18, **caractérisée en ce que** la séquence nucléotidique de transférase de galactosyle est une séquence nucléotidique de transférase de beta-1,4 galactosyle (beta-1,4 galT).

20. Une méthode selon la revendication 19, **caractérisée en ce que** la séquence nucléotidique de transférase de galactosyle est la séquence nucléotidique de transférase de beta-1,4 galactosyle (beta-1,4 galT) humaine.

21. Une méthode selon une des revendications 15 à 20, **caractérisée en ce que** le polypeptide glycosylé est choisi dans un groupe comprenant une protéine ayant une séquence primaire en acides aminés d'une protéine humaine, une séquence primaire en acides aminés d'une protéine de mammifère non humain, une séquence primaire en acides aminés d'un anticorps ou d'un fragment actif de celui-ci, et/ou une séquence primaire en acides aminés d'une protéine de non mammifère.

22. Une méthode selon une des revendications 15 à 21, **caractérisée en ce que** le polypeptide glycosylé est choisi dans le groupe constitué de l'insuline humaine, la préproinsuline, le VEGF, la proinsuline, le glucagon, les interférons tel que l'alpha-interféron, le béta-interféron, le gamma-interféron, les facteurs de la coagulation du sang choisis parmi les facteurs VII, VIII, IX, X, et XII, les hormones de la fertilité incluant l'hormone lutéinisante, l'hormone de stimulation des follicules, les facteurs de croissance incluant le facteur de croissance de l'épiderme, le facteur de croissance dérivé des plaquettes, le facteur de stimulation des colonies de granulocytes, la prolactine, l'oxytocine, l'hormone de stimulation de la thyroïde, l'hormone adrénocorticotropique, la calcitonine, l'hormone parathyroïde, la somatostatine, l'érythropoïétine (EPO), et des enzymes telle que la beta-glucocérébrosidase, l'hémoglobine, l'albumine sérique, et le collagène, et des protéines humaines et non humaines choisies parmi les amidases, les amylases, les carbohydrases, la cellulase, la dextranase, les estérases, les glucanases, la glucoamylase, la lactase, les lipases, la pepsine, les peptidases, les phytases, les protéases, les pectinases, la caséine, les protéines de lactosérum, les protéines de colza, le gluten et l'albumine d'oeuf.

23. Une méthode selon une des revendications 14 à 22, **caractérisée en ce que** la cellule de bryophyte est choisie parmi les espèces des genres *Physcomitrella, Funaria, Sphagum, Ceratodon, Marchantia* et *Sphaerocarpos.*

24. Une méthode selon la revendication 23, **caractérisée en ce que** la cellule de bryophyte est choisie dans *Physcomitrella.*

25. Une méthode selon la revendication 24, **caractérisée en ce que** la cellule de bryophyte est de *Physcomitrella pat ens.*

26. Une méthode selon une des revendications 15 à 25, **caractérisée en ce que** le polypeptide glycosylé est choisi dans un groupe comprenant une protéine ayant une séquence primaire en acides aminés d'une protéine humaine, une séquence primaire en acides aminés d'une protéine de mammifère non humain, une séquence primaire en acides aminés d'un anticorps ou d'un fragment actif de celui-ci, et/ou une séquence primaire en acides aminés d'une protéine de non mammifère.

27. Une méthode selon la revendication 26, **caractérisée en ce que** le polypeptide glycosylé est un polypeptide humain.

28. Une méthode selon la revendication 26 ou 27, **caractérisée en ce que** le polypeptide glycosylé est choisi dans le groupe constitué de l'insuline humaine, la préproinsuline, le VEGF, la proinsuline, le glucagon, les interférons tel que l'alpha-interféron, le béta-interféron, le gamma-interféron, les facteurs de la coagulation du sang choisis parmi les facteurs VII, VIII, IX, X, et XII, les hormones de la fertilité incluant l'hormone lutéinisante, l'hormone de stimulation des follicules, les facteurs de croissance incluant le facteur de croissance de l'épiderme, le facteur de croissance dérivé des plaquettes, le facteur de stimulation des colonies de granulocytes, la prolactine, l'oxytocine, l'hormone de stimulation de la thyroïde, l'hormone adrénocorticotropique, la calcitonine, l'hormone parathyroïde, la somatostatine, l'érythropoïétine (EPO), et des enzymes telle que la beta-glucocérébrosidase, l'hémoglobine, l'albumine sérique, et le collagène.

29. Une méthode selon une des revendications 15 à 28, **caractérisée en ce que** le promoteur exogène est choisi parmi les promoteurs inductibles, régulés par des produits chimiques, constitutifs ou spécifiques de type cellulaire.

30. Une collection de vecteurs d'acide nucléique appropriés pour produire au moins une cellule de bryophyte transformée comprenant un double knock-out de la transférase de fucosyle et la transférase de xylosyle, **caractérisée en ce qu'** elle contient des glycanes liés par N (N-Linked) modifiés sans résidus fucosyle 1,3-Linked et xylosyle 1,2-Linked détectables, la dite collection de vecteurs d'acide nucléique comprenant i) une première séquence d'acide nucléique qui est spécifiquement ciblée vers une séquence nucléotidique endogène de la transférase de fucosyle, et ii) une seconde séquence d'acide nucléique qui est spécifiquement ciblée vers une séquence nucléotidique endogène de la transférase de xylosyle.

31. Une collection de vecteurs d'acide nucléique selon la revendication 30, incluant de plus un polynucléotide codant pour un polypeptide glycosylé.

32. Une collection de vecteurs d'acide nucléique selon la revendication 30 ou 31, incluant de plus un polynucléotide codant pour une transférase de glycosyle de mammifère fonctionnelle, pour être utilisée dans une méthode selon une des revendications 15 à 29.

33. Une collection de vecteurs d'acide nucléique selon la revendication 32, **caractérisée en ce que** le dit polynucléotide code pour une transférase de galactosyle de mammifère.

34. Une collection de vecteurs d'acide nucléique selon la revendication 33, **caractérisée en ce que** le dit polynucléotide code pour une transférase de beta-1,4 galactosyle humaine.

35. Une cellule hôte contenant la collection de vecteurs d'acide nucléique selon une des revendications 30 à 34.

36. Une cellule hôte selon la revendication 35 qui est une cellule de bryophyte.

37. Une cellule hôte selon la revendication 35 qui est une cellule procaryote.

38. Une méthode de production d'une cellule hôte selon une des revendications 35 à 37, la méthode incluant l'incorporation de la dite collection de vecteurs d'acide nucléique dans la cellule au moyen d'une transformation.

39. L'utilisation d'une collection de vecteurs d'acide nucléique selon une des revendications 30 à 34 dans la production d'une cellule de bryophyte transgénique.

40. Une cellule hôte selon la revendication 35 ou 36 qui est comprise dans un bryophyte ou une partie de bryophyte ou dans un extrait ou un dérivé de bryophyte ou dans une culture de cellule de bryophyte.

41. Un plant de bryophyte ou un tissu de bryophyte comprenant une cellule de bryophyte selon une des revendications 1 à 13, 36 et 40.

42. Une méthode de production d'un plant de bryophyte, la méthode incluant l'incorporation de la collection de vecteurs d'acide nucléique selon une des revendications 30 à 34 dans une cellule de bryophyte et la régénération d'un bryophyte de la dite cellule.
